# EUROPÄISCHE PATENTSCHRIFT

(11) **EP 2 943 273 B1**
(45) Veröffentlichungstag und Bekanntmachung des Hinweises auf die Patenterteilung: **19.04.2017**
(21) Anmeldenummer: 14700042.6
(22) Anmeldetag: 03.01.2014
(51) Int. Cl.: B01J 8/22, B01J 8/18, B01J 4/00, B01J 19/24, C07C 209/36

(54) **VORRICHTUNG UND VERFAHREN ZUR KONTINUIERLICHEN UMSETZUNG VON FLÜSSIGKEITEN MIT GASEN**
APPARATUS AND METHOD FOR THE CONTINUOUS INTRODUCTION OF GASES INTO LIQUIDS
DISPOSITIF ET PROCÉDÉ DESTINÉS À LA CONVERSION CONTINUE DE LIQUIDES AVEC DES GAZ

(30) Priorität: 11.01.2013 EP 13150920
(43) Veröffentlichungstag der Anmeldung: 18.11.2015
(73) Patentinhaber: BASF SE, 67056 Ludwigshafen am Rhein (DE)
(72) Erfinder: BEY, Oliver, 67150 Niederkirchen (DE); KUNKELMANN, Christian, 64367 Mühltal (DE); BAUER, Roland, 67591 Offstein (DE); RAICHLE, Andreas, 67063 Ludwigshafen (DE); RIECHERS, Hartmut, 67435 Neustadt (DE); ZEHNER, Peter, 67273 Weisenheim am Berg (DE)
(86) Internationale Anmeldenummer: PCT/EP2014/050046
(87) Internationale Veröffentlichungsnummer: WO 2014/108353

(56) Entgegenhaltungen:
- EP-A1- 0 130 499
- EP-A2- 0 034 739
- WO-A1-2010/076251
- DE-A1- 19 854 637
- DE-A1-102008 041 652
- GB-A- 1 013 888

## Beschreibung

Die vorliegende Erfindung betrifft eine Vorrichtung des Typs Loop-Venturi-Reaktor für die kontinuierliche Umsetzung von Flüssigkeiten mit Gasen, insbesondere für Hydrierungen, Oxidationen oder Acetylierungen, z.B. für die Herstellung von Toluylendiamin durch Hydrierung von Dinitrotoluol sowie ein Verfahren zur kontinuierlichen Umsetzung von flüssigen Reaktanden mit gasförmigen Reaktanden in der Vorrichtung. In der erfindungsgemäßen Vorrichtung erfolgt die Umlenkung einer internen Zirkulationsströmung im Reaktor mittels einer Umlenkwanne, die unterhalb eines Wärmeübertragers angeordnet ist.

In vielen chemischen Verfahren sind der Gas-Flüssigkeits-Stoffübergang und die Wärmeabführleistung die geschwindigkeitsbestimmenden Schritte. So wird bei der in der Technik üblichen Herstellung von aromatischen Mono-und/oder Polyaminen durch Umsetzung der entsprechenden Nitroverbindungen mit Wasserstoff eine beträchtliche Wärmemenge frei. Gleiches gilt für viele andere Hydrierungen, Oxidationen oder Acetylierungen. Maßnahmen zur Verbesserung der Wärmeabfuhrleistung sind aus dem Stand der Technik an sich bekannt.

In EP-A-634 391 wird ein Verfahren zur Hydrierung von aromatischen Polynitroverbindungen beschrieben, in dem ein Loop-Venturi-Reaktor mit einem Ejektor verwendet wird (Schlaufenreaktor mit Venturi-Düse). Die Verfahrensführung beruht auf speziellen Bedingungen wie das genaue Umwälzvolumenverhältnis, der Energieeintrag, einem genau eingestellten Wasserstoffvolumenstrom, womit einerseits Nebenprodukte vermieden werden sollen und andererseits die freiwerdende Wärme zur Dampferzeugung genutzt werden kann. Bei diesem Verfahren kann es, bedingt durch die Anordnung eines Wärmeübertragers zur Abführung der Reaktionswärme außerhalb des Schlaufenreaktors, im Ejektor und im Reaktor zu örtlichen Überhitzungen mit sofortigem Einsetzen von Nebenreaktionen wie Kernhydrierungen, hydrogenolytischen Spaltungen bzw. Bildung von hochmolekularen, teerartigen Produkten, die die Katalysatoroberfläche belegen, kommen. Darüber hinaus stellt sich im Reaktorvolumen außerhalb des Ejektors eine bezüglich des Strömungs- und Verweilzeitverhaltens reine Blasensäulencharakteristik ein, in dem regellose klein-und großräumige Wirbel mit vergleichsweise geringer Stoffübergangsleistung auftreten. Eine wesentliche Verbesserung der Hydrierausbeute, der Hydrierselektivität und der Raum-Zeit-Ausbeute wird somit bei diesem Verfahren kaum erreicht. Außerdem wird auch hier durch das Umpumpen der gesamten Reaktionsmischung der Katalysator mechanisch stark beansprucht, was wiederum zu einer verminderten Standzeit des Katalysators führt.

Als ein für die Abführung der Reaktionswärme besonders geeigneter Reaktor wurde daher in WO 00/35852 ein Reaktor mit interner und externer Zirkulationsströmung (sogenannte interne und externe Schlaufe) vorgeschlagen, der als vertikal aufrecht stehender Apparat ausgebildet ist, mit einer Treibstrahldüse an seinem oberen Ende, über die in den oberen Bereich des Reaktors das aus dem Reaktorsumpf abgezogene Reaktionsgemisch über die externe Schlaufe eingedüst wird, und das anschließend in ein zentrales Einsteckrohr, welches in Reaktorlängsrichtung angeordnet ist, einströmt, dieses von oben nach unten durchströmt und in einer internen Schlaufenbewegung außerhalb des Einsteckrohrs erneut nach oben strömt. Für die Abführung der Reaktionswärme werden im Reaktorinnenraum Fieldrohre vorgeschlagen. Fieldrohr-Wärmeübertrager bezeichnen in bekannter Weise Wärmeübertrager, die ein Bündel paralleler Doppelmantelrohre aufweisen, wobei die in den Reaktorraum ragenden Enden der Außenrohre geschlossen und die entsprechenden Enden der Innenrohre offen sind, dergestalt, dass das Kühlmedium über einen außerhalb des Reaktorraums angeordneten Zuführraum in die Innenrohre einströmt und über den Raum zwischen Innen- und Außenrohren sowie einen Abführraum ausströmt. Sie sind durch ein hohes Verhältnis von Wärmeübertragerfläche zu Volumen des Reaktionsraumes gekennzeichnet und sind somit besonders für die Abfuhr der freiwerdenden Reaktionswärme geeignet.

Die EP-A 1140349 offenbart einen Reaktor für Gas-Flüssig- oder Gas-Flüssig-Fest-Reaktionen in hochzylindrischer Bauform mit einer im oberen Reaktorbereich angeordneten, nach unten gerichteten Strahldüse, über die die Edukte und das Reaktionsgemisch zugeführt werden sowie mit einem Abzug im unteren Reaktorbereich, über den das Reaktionsgemisch entnommen und über einen äußeren Kreislauf mittels einer Pumpe der Strahldüse erneut zugeführt wird. In dem Reaktor ist ein konzentrisches Leitrohr angeordnet, das sich im Wesentlichen über die gesamte Länge des Reaktors mit Ausnahme der Reaktorenden erstreckt und eine Querschnittsfläche im Bereich von einem Zehntel bis zur Hälfte der Querschnittsfläche des Reaktors aufweist. Es hat sich allerdings gezeigt, dass in den Reaktoren nach dem Stand der Technik, insbesondere in der Strömung unterhalb der internen Schlaufe beim Übergang zur externen Schlaufe, d. h. in der Nähe des Reaktorausgangs, erhöhte Gehalte an Nitroaromaten auftreten können.

Weitere Reaktoren der eingangs genannten Art sind aus DE 10 2008 041 652 A1 bekannt. Die vorgenannten Reaktoren und entsprechende Verfahren nach dem Stand der Technik haben insbesondere den Nachteil, dass sie bei einer Auslegung für große umgewälzte Volumina Kurzschlussströme hervorrufen, die in unerwünschter Weise dafür sorgen, dass der Strom aus dem Reaktor in die externe Zirkulationsströmung gelangt, ohne eine für die Umsetzung notwendige Strecke im Reaktor durchlaufen zu haben. Hierdurch sinkt die Konzentration des flüssigen Reaktanden nicht ausreichend ab, bevor es aus dem Reaktor gelangt.

Es war die Aufgabe der vorliegenden Erfindung, die vorgenannten Nachteile zu vermeiden. Es sollte insbesondere eine Vorrichtung zur Durchführung von Gas-Flüssig-Reaktionen aufgefunden werden, welche Kurzschlussströme im unteren Bereich des Reaktors vermeidet und mit der besonders effektiv möglichst geringe Produktkonzentrationen am Auslass zur Entnahme des Reaktionsmediums aus dem Reaktor erhalten werden.

Diese Aufgabe wird gelöst durch eine Vorrichtung zur kontinuierlichen Umsetzung von Flüssigkeiten mit Gasen, insbesondere zur Herstellung von Toluylendiamin durch Hydrierung von Dinitrotoluol, gemäß Anspruch 1.

Diese Aufgabe wird außerdem gelöst durch ein Verfahren zur kontinuierlichen Umsetzung von flüssigen Reaktanden mit gasförmigen Reaktanden in der vorgenannten Vorrichtung.

Der Reaktor der erfindungsgemäßen Vorrichtung umfasst einen abgegrenzten Raum (definiert durch ein Behältnis) und ist dafür ausgelegt, um unter definierten Bedingungen bestimmte Reaktionen ablaufen zu lassen und steuern zu können. Der Begriff "Reaktor" umfasst sämtliche Bereiche innerhalb des abgegrenzten Raums, d.h. den Reaktionsraum, den Gasraum und den Raum, der durch das Kühlmedium eingenommen wird (Kühlbereich).

Unter einem vertikal ausgedehnten Reaktor wird ein Reaktor verstanden, der in vertikaler Richtung (Längsrichtung des Reaktors) eine größere Ausdehnung aufweist als in horizontaler Richtung (Querrichtung des Reaktors). Der Reaktor ist im Betrieb vertikal aufrechtstehend. Unter "im Betrieb" ist bei Durchführung des erfindungsgemäßen Verfahrens zu verstehen.

Im Rahmen der vorliegenden Erfindung beziehen sich die Bezeichnungen oben, unten, neben, oberhalb und unterhalb auf die vertikale Richtung (Längsrichtung) des Reaktors, d. h. sie beziehen sich auf eine ausgedehnte Anordnung.

Der Reaktionsraum des Reaktors ist der Raum innerhalb des Reaktors, der zur Aufnahme des Reaktionsmediums vorgesehen ist und umfasst demzufolge alle Bereiche innerhalb des Reaktors, die im Betrieb mit dem Reaktionsmedium in Fluidverbindung stehen.
Fluidverbindung ist die räumliche Verbindung innerhalb des Reaktors zweier beliebiger Stellen oder Elemente, über die ein Fluid, also insbesondere das Reaktionsmedium oder das Kühlmedium, von einer Stelle oder von einem Element zur anderen Stelle oder zum anderen Element gelangen kann.

Ein Reaktionsraum kennzeichnet insbesondere einen Bereich innerhalb des Reaktors, in welchem im Betrieb die Umsetzung abläuft und in dem Mittel zur Kühlung vorgesehen sind. Hierdurch kann die Umsetzung im Reaktionsraum im Wesentlichen ohne signifikante Temperaturänderungen ablaufen, d. h. im Wesentlichen isotherm.
Die erfindungsgemäße Vorrichtung umfasst mindestens ein Mittel zur Umlenkung der internen Zirkulationsströmung, wobei das mindestens eine Mittel innerhalb des Reaktors und unterhalb des Wärmeübertragers angebracht ist. Das genannte Mittel ist außerdem oberhalb des Reaktorbodens angeordnet. Erfindungsgemäß ist das Mittel zur Umlenkung des Stromes des Reaktionsmediums eine Umlenkwanne.
Eine Umlenkwanne ist dadurch gekennzeichnet, dass sie eine in Querrichtung des Reaktors ausgerichtete Fläche sowie eine seitliche, sich in Längsrichtung des Reaktors erstreckende Umrandung aufweist, die nach oben, d.h. in Richtung des Ejektors zeigt. Eine Umlenkwanne ist als Mittel zur Umlenkung der internen Zirkulationsströmung von dem aus dem Stand der Technik bekannten Prallblech zu unterscheiden. Ein Prallblech ist dadurch gekennzeichnet, dass es eine in Querrichtung des Reaktors ausgerichtete Fläche sowie keine seitliche, sich in Längsrichtung des Reaktors erstreckende Umrandung aufweist. Durch Verwendung einer Umlenkwanne ist es möglich, Kurzschlussströme im unteren Bereich des Reaktors zu vermeiden und besonders effektiv möglichst geringe Produktkonzentrationen am Auslass zur Entnahme des Reaktionsmediums aus dem Reaktor einzustellen.

Die Umlenkwanne erstreckt sich in der horizontalen Ebene in Querrichtung des Reaktors über einen Großteil der Fläche, die von der unteren Seite des Wärmeübertragers im Querschnitt des Reaktors eingenommen wird, jedoch nicht über den gesamten Querschnitt des Wärmeübertragers. Hierdurch wird erreicht, dass das Reaktionsmedium im äußeren Bereich des Reaktors nur dann zu dem Auslass zur Entnahme des Reaktionsmediums aus dem Reaktor gelangen kann, nachdem es in den Rohren oberhalb der Umlenkwanne nach oben geströmt ist. Bevorzugt strömt das Reaktionsmedium in den Rohren oberhalb der Umlenkwanne nach oben und ein Teilstrom dann in den Rohren außerhalb des Querschnitts der Umlenkwanne wieder nach unten.

Vorzugsweise beträgt die horizontale Querschnittsfläche der Umlenkwanne von 60 bis 98 % der Fläche, die durch das untere Ende des Wärmetauschers im Querschnitt des Reaktors eingenommen wird, insbesondere von 70 bis 95 %, besonders bevorzugt von 75 bis 90 %.
Die Höhe der Umrandung der Umlenkwanne beträgt von 1 bis 30 % des längsten Durchmessers der Umlenkwanne, insbesondere von 2 bis 20 %, besonders bevorzugt von 3 bis 15 %. Der Winkel der Umrandung relativ zur Innenfläche der Umlenkwanne beträgt vorzugsweise von 90 bis 170°, insbesondere von 90 bis 150°, besonders bevorzugt von 90 bis 120°.
In einer bevorzugten Ausführungsform weist die Umlenkwanne Entleerungsöffnungen auf, die insbesondere zumindest teilweise verschlossen werden können. Im Betrieb sind die Öffnungen zumindest teilweise oder bevorzugt ganz geschlossen, wohingegen sie beim Stillstand geöffnet werden können.

Wärmeübertrager ist ein Mittel zur Übertragung von Wärme an ein Kühlmedium zwecks Abfuhr von freiwerdender Reaktionswärme. Als Wärmeübertrager kommen grundsätzlich verschiedene Typen in Betracht, sofern sie die benötigte Wärmeübertarungsfläche aufweisen. Bevorzugte Wärmeübertrager sind Rohrbündel-Wärmeübertrager, Plattenwärmeübertrager, Fieldrohr-Wärmeübertrager und Rohrschlangen-Wärmeübertrager.
Der Wärmeübertrager befindet sich innerhalb des Reaktors und bedingt die Wärmeübertragungsfläche zwischen dem Reaktionsraum und dem Kühlbereich. Vorzugsweise umfasst der Wärmeübertrager Wärmeübertragerrohre und ist insbesondere ein Rohrbündelwärmeübertrager. In einer besonders bevorzugten Ausführungsform steht der die Wärmeübertragerrohre umgebende Bereich mit dem Einlass zur Zuführung des Kühlmediums und dem Auslass zur Entnahme des Kühlmediums in Fluidverbindung, und der innere Bereich der Wärmeübertragerrohre bildet einen Teil des Reaktionsraums und steht mit der mindestens einen Treibstrahldüse und dem Auslass zur Entnahme des Reaktionsmediums in Fluidverbindung.
Wärmeübertragerrohre sind dabei an den Enden mit Öffnungen versehene Hohlkörper, die insbesondere länglich ausgedehnt sind, d. h. eine größere Länge als einen Innendurchmesser aufweisen. Insbesondere beträgt das Verhältnis von Länge zu Innendurchmesser mindestens 10, bevorzugt mindestens 20, besonders bevorzugt mindestens 50, insbesondere mindestens 100. Die Wärmeübertragerrohre grenzen zudem das Reaktionsmedium von dem Kühlmedium ab und erlauben die Übertragung von Wärme zwischen den beiden Medien. Vorzugsweise ist der Querschnitt der Rohre im Wesentlichen kreisförmig. Es ist jedoch grundsätzlich möglich, von der Kreisform abzuweichen, um besondere Strömungsführungen zu realisieren.

Der Durchmesser eines einzelnen Wärmeübertragerrohres beträgt vorzugsweise von 10 bis 100 mm, insbesondere von 20 bis 50 mm. Die Wärmeübertragerrohre sind vorzugsweise vertikal im Reaktor angebracht, d. h. in Längsrichtung des Reaktors.

Unter Rohrbündel werden im Rahmen der vorliegenden Erfindung mindestens zwei parallel angeordnete Wärmeübertragerrohre verstanden. Rohrbündel sind für die Aufnahme großer Wärmemengen besonders geeignet. Der Fachmann wählt die Zahl der Rohre im Rohrbündel entsprechend der notwendigen Wärmeübertragungsfläche bezogen auf das Volumen des Reaktionsraumes. Die Zahl der Wärmeübertragerrohre im Reaktor beträgt vorzugsweise mindestens 100, insbesondere mindestens 200, bevorzugt mindestens 300, ganz besonders bevorzugt mindestens 500. Außerdem beträgt die Zahl der Wärmeübertragerrohre im Reaktor vorzugsweise höchstens 10.000, insbesondere höchstens 7.000, besonders bevorzugt höchstens 5.000, ganz besonders bevorzugt höchstens 4.000 .

Das Rohrbündel ist dabei vorzugsweise innerhalb des vertikal ausgedehnten Reaktors um die mindestens eine Mischkammer und, sofern vorhanden, um den mindestens einen Diffusor herum angebracht (d. h. das Rohrbündel umgibt die Mischkammer und ggf. den Diffusor in vertikaler Richtung vollständig) und nimmt vorzugsweise mindestens 20 Flächen-%, insbesondere mindestens 30 Flächen-%, besonders bevorzugt mindestens 40 Flächen.-%, ganz besonders bevorzugt mindestens 50 Flächen-% des inneren horizontalen Querschnitts des Reaktors ein.

Die Zahl der Rohre im Rohrbündel beträgt dabei vorzugsweise von 100 bis 10.000, besonders bevorzugt von 500 bis 5.000. Der Fachmann wählt Zahl, Länge und Durchmesser der Wärmeübertragerrohre insbesondere in Abhängigkeit von der pro Zeiteinheit und pro Volumen frei werdenden Reaktionswärme, welche die zur Wärmeabfuhr erforderliche Temperaturdifferenz und Wärmeübertragungsfläche bestimmt.

Das Verhältnis der Anzahl an Wärmeübertragerrohren zur Anzahl an Ejektoren im Reaktor beträgt vorzugsweise mindestens 100, insbesondere mindestens 200, besonders bevorzugt mindestens 500. Das genannte Verhältnis von Wärmeübertragerrohren zu Ejektor beträgt vorzugsweise höchstens 10.000, insbesondere höchstens 7.500, besonders bevorzugt höchstens 5.000.

Durch die vorgenannten Maßnahmen in Bezug auf die Zahl der Wärmeübertragerrohre im Reaktor ist gewährleistet, dass die interne Zirkulationsströmung mit einer Vielzahl von Wärmeübertragerrohren in Fluidverbindung steht. Im Bereich der vorgenannten Verhältnisse wird im Betrieb eine günstige interne Zirkulationsströmung erzielt und gleichzeitig eine hohe Wärmemenge übertragen.

In einer besonders bevorzugten Ausführungsform steht der die Wärmeübertragerrohre umgebende Bereich mit dem Einlass zur Zuführung des Kühlmediums und dem Auslass zur Entnahme des Kühlmediums in Fluidverbindung, und der innere Bereich der Wärmeübertragerrohre bildet einen Teil des Reaktionsraums und steht mit der mindestens einen Treibstrahldüse und dem Auslass zur Entnahme des Reaktionsmediums aus dem Reaktor in Fluidverbindung.

Die Wärmetauscherrohre sind dabei insbesondere dergestalt in Fluidverbindung mit der mindestens einen Treibstrahldüse und dem Auslass zur Entnahme des Reaktionsmediums aus dem Reaktor, dass im Betrieb die Reaktionsmischung zunächst die Treibstrahldüse, dann die Mischkammer und den Diffusor, anschließend die Wärmetauscherrohre und schließlich den Auslass zur Entnahme des Reaktionsmediums aus dem Reaktor durchströmt.

In dieser besonders bevorzugten Ausführungsform ist der Reaktor so konzipiert, dass im Betrieb das Kühlmedium die Wärmeübertragerrohre umgibt und das Reaktionsmedium durch das Innere der Wärmeübertragerrohre strömt. Der Begriff Reaktionsmedium kennzeichnet die Mischung der Edukte und Produkte im Reaktor, d. h. die Edukte, die reagierende Mischung und/oder das Reaktionsprodukt. Das Reaktionsmedium ist insbesondere eine Mehrphasenmischung aus Reaktionsflüssigkeit, dispergiertem Gas und ggf. suspendiertem festen Katalysator.

Dies wird in dieser besonders bevorzugten Ausführungsform dadurch erreicht, dass der die Wärmeübertragerrohre umgebende Bereich mit dem Einlass zur Zuführung des Kühlmediums und dem Auslass zur Entnahme des Kühlmediums in Fluidverbindung steht, so dass beim Betrieb des Reaktors die Wärmeübertragerrohre vom Kühlmedium umgeben sind. Gleichzeitig ist der innere Bereich der Wärmeübertragerrohre ein Bestandteil desReaktionsraums und steht mit der mindestens einen Treibstrahldüse und dem mindestens einen Auslass zur Entnahme des Reaktionsmediums in Fluidverbindung, so dass beim Betrieb des Reaktors das Reaktionsmedium die Wärmeübertragerrohre innen durchströmt.

In dieser besonders bevorzugten Ausführungsform ist somit der die Wärmeübertragerrohre umgebende Bereich mit dem Einlass zur Zuführung des Kühlmediums und dem Auslass zur Entnahme des Kühlmediums so verbunden, dass beim Betrieb des Reaktors die Wärmeübertragerrohre vom Kühlmedium umgeben sind. In dieser besonders bevorzugten Ausführungsform ist somit zudem der innere Bereich der Wärmeübertragerrohre mit den Treibstrahldüsen und dem Auslass zur Entnahme des Reaktionsmediums so verbunden dass beim Betrieb des Reaktors das Reaktionsmedium die Wärmeübertragerrohre innen durchfließt.

Hierdurch werden bei großen frei werdenden Wärmemengen lokale Temperaturerhöhungen vermieden und die Raumausnutzung des Reaktorvolumens bzw. die Wärmeabfuhr und die Verweilzeitverteilung im Reaktor optimiert. Insbesondere können so eine große Zahl an zueinander in großer räumlicher Nähe befindlichen Wärmeübertragerrohren realisiert werden, ohne dass es (wie z.B. bei einer Verwendung von Fieldrohren) zu verengten Strömungswegen zwischen den Rohren kommt, die andernfalls zu lokalen Termperaturerhöhungen durch räumlich inhomogene Wärmeübertragung führen.

Vorzugsweise beträgt die Wärmeübertragungsfläche bezogen auf das Reaktionsvolumen von 20 bis 400 m² / m³, insbesondere von 25 bis 300 m² / m³, besonders bevorzugt von 40 bis 100 m² / m³.

Die erfindungsgemäße Vorrichtung umfasst im Betrieb zudem mindestens einen Gasraum im oberen Bereich des Reaktors. Der Gasraum im oberen Bereich des Reaktors ist der Raum innerhalb des Reaktors, welcher oberhalb des Flüssigkeitsspiegels des Reaktionsmediums angeordnet ist. Dementsprechend ist der Gasraum im Betrieb mit den gasförmigen Reaktanden und ggf. Inertgas gefüllt.

Die erfindungsgemäße Vorrichtung umfasst zudem mindestens einen Einlass zur Zuführung des Kühlmediums in den Wärmeübertrager.

Unter Einlass wird im Rahmen der vorliegenden Erfindung jedes Mittel verstanden, das zum Einlass des entsprechenden Mediums vorgesehen ist. Entsprechend wird unter Auslass im Rahmen der vorliegenden Erfindung jedes Mittel verstanden, das zum Auslass des entsprechenden Mediums vorgesehen ist. Die vorgenannten Mittel können insbesondere Öffnungen, Rohre, Ventile oder Düsen sein.

Die erfindungsgemäße Vorrichtung umfasst zudem mindestens einen Auslass zur Entnahme des Kühlmediums aus dem Wärmeübertrager. Der Einlass zur Zuführung des Kühlmediums in den Wärmeübertrager ist vorzugsweise im unteren Bereich des Reaktors, in vertikaler Richtung oberhalb des unteren Endes des Reaktionsraumes und unterhalb des Auslasses für die Entnahme des Kühlmediums angebracht. In diesem Fall strömt das Kühlmedium im Wärmeübertrager von unten nach oben.

Der Auslass ist vorzugsweise in vertikaler Richtung auf der Höhe unterhalb des oberen Endes des Reaktionsraumes angebracht.

Die erfindungsgemäße Vorrichtung umfasst zudem mindestens einen Einlass zur Zuführung des gasförmigen Reaktanden in den Reaktor. Vorzugsweise ist der Einlass zur Zuführung des gasförmigen Reaktanden in vertikaler Richtung auf Höhe unterhalb des Wärmeübertragers, jedoch oberhalb des Mittels zur Umlenkung des nach unten durch die Mischkammer strömenden Reaktionsmediums angebracht. Da die genannte Umlenkung dergestalt erfolgt, dass das Reaktionsmedium durch den Wärmeübertrager wieder nach oben strömt, ergibt sich im Betrieb der Vorrichtung eine interne Zirkulationsströmung, weshalb auch von Umlenkung einer internen Zirkulationsströmung gesprochen werden kann.

Durch die Anbringung unterhalb des Wärmübertragers ist es möglich, den Energieverbrauch zur Einbringung des gasförmigen Reaktanden zu minimieren, da die interne Zirkulationsströmung für die Durchmischung optimal genutzt und durch den Auftrieb zusätzlich verstärkt werden kann.

Um eine schnelle und gleichmäßige Durchmischung des Reaktionsmediums im Betrieb zu erreichen, ist es vorteilhaft, den gasförmigen Reaktanden in einem horizontalen Querschnitt des Reaktionsraums unterhalb der Wärmeübertragerrohre auf einen Teil, vorzugsweise mindestens 50 %, insbesondere mindestens 70 %, besonders bevorzugt mindestens 80 % der Querschnittsfläche zu verteilen. Grundsätzlich ist hierfür jedes Mittel geeignet, welches ausreichend viele und geeignet angeordnete Austrittsöffnungen aufweist. Von Bedeutung ist außerdem der Querschnitt der Austrittsöffnung. Diesbezüglich vorteilhaft sind Austrittsöffnungen mit einem Durchmesser von 1 bis 20 mm, insbesondere von 3 bis 10 mm. Diesbezüglich vorteilhaft ist außerdem eine Geschwindigkeit in den Austrittsöffnungen von 5 bis 300 m/s, insbesondere von 10 bis 200 m/s.

Wegen der Verwendung eines Rohrbündels als Wärmeübertrager ist eine spezifisch auf den Wärmeübertrager angepasste Ausführungsform bevorzugt, da sich das Reaktionsmedium im Betrieb im inneren Bereich der Rohre befindet.

In einer alternativen Ausführungsform erfolgt der Eintrag des gasförmigen Reaktanden von oben, d. h. der Einlass zur Zuführung des gasförmigen Reaktanden befindet sich oberhalb des Wärmeübertragers, bevorzugt in dem Gasraum. Nachteilig ist hierbei, dass mehr Energie aufgewendet werden muss, um den gasförmigen Reaktanden einzubringen. Vorteilhaft ist jedoch, dass die gleichmäßige Verteilung des gasförmigen Reaktanden im Reaktionsraum besonders einfach möglich ist. Die oben beschriebene Zuführung unterhalb des unteren Endes der Wärmeübertragerrohre und oberhalb des Mittels zur Umlenkung der internen Zirkulationsströmung ist jedoch bevorzugt.

In einer besonders bevorzugten alternativen Ausführungsform ist zusätzlich zu der vorgenannten Ausführungsform der Zuführung oberhalb des Wärmeübertragers ein weiterer Einlass zur Zuführung des gasförmigen Reaktanden unterhalb des unteren Endes der Wärmeübertragerrohre und oberhalb des Mittels zur Umlenkung der internen Zirkulationsströmung vorgesehen. Hierdurch lässt sich eine einfache Verteilung des gasförmigen Reaktanden mit geringem energetischen Aufwand für die Zuführung realisieren.
Die erfindungsgemäße Vorrichtung umfasst zudem mindestens einen Einlass zur Zuführung des flüssigen Reaktanden in den Reaktor. Vorzugsweise ist der mindestens eine Einlass zur Zuführung des flüssigen Reaktanden mindestens ein oberhalb der Mischkammer, insbesondere im Gasraum, angebrachtes Rohr. In einer alternativen Ausführungsform kann eine Flüssigkeitsdüse verwendet werden. Der Einlass für den flüssigen Reaktanden wird vorzugsweise in unmittelbarer räumlicher Nähe zu der Treibstrahldüse angebracht. Hierdurch ist eine schnellere und vollständigere Durchmischung des flüssigen Reaktanden mit dem Reaktionsmedium möglich.

Die Zuführung für den flüssigen Reaktanden kann bis zum Einlass gekühlt werden, vorzugsweise innerhalb des Reaktors, um zu verhindern, dass thermisch empfindliche Reaktanden zu stark erhitzt werden. Dies ist insbesondere bei der Hydrierung von Dinitrotoluol von Vorteil. In diesem Fall wird auf Temperaturen von 60 bis 150 °C, bevorzugt von 70 bis 110 °C temperiert. Besonders bevorzugt ist die Zuführung des Dinitrotoluols von zwei ineinanderliegenden Mantelrohren umgegeben: Im inneren Mantelrohr strömt das Kühlmedium zur Spitze der Zuführung und im äußeren Mantelrohr wieder nach außen zurück. Vorzugsweise erfolgt der Einlass des flüssigen Reaktanden so, dass im Betrieb keine unmittelbare räumliche Nähe zum Reaktionsmedium besteht.

Die erfindungsgemäße Vorrichtung umfasst außerdem mindestens eine nach unten gerichtete Treibstrahldüse, die vertikal im Gasraum des Reaktors angeordnet ist. Durch die nach unten gerichtete Anbringung ist es möglich, selbsttätig, d. h. durch die Treibstrahldüse, den gasförmigen Reaktanden im Gasraum anzusaugen. Die Treibstrahldüse wird mit Flüssigkeit (dem Reaktionsmedium) betrieben und ist somit eine Flüssigkeitsdüse.

Die Treibstrahldüse oder die Treibstrahldüsen können jeweils als Einlochdüse oder als Mehrlochdüse ausgestaltet sein. Eine Einlochdüse weist eine Düsenunterseite mit genau einer Öffnung, die sogenannte Mündung, auf. Eine Mehrlochdüse weist entsprechend eine Düsenunterseite mit mehreren Öffnungen auf.

Eine Einlochdüse ist einfach herzustellen. Eine Mehrlochdüse ermöglicht eine besonders effektive Ansaugung der gasförmigen Reaktanden, so dass der Flüssigkeitsspiegel auf minimalen Abstand zu der Treibstrahldüse gehalten werden kann. Werden die verschiedenen Mündungen einer Mehrlochdüse auf unterschiedlichen horizontalen Ebenen angeordnet, kann der Flüssigkeitsspiegel besonders stabil auf minimalem Abstand zu der Treibstrahldüse gehalten werden.

Mehrlochdüsen mit rundem Querschnitt der Öffnungen und einem Durchmesser der Öffnungen von 5 bis 100 mm, vorzugsweise von 10 bis 80 mm, besonders bevorzugt von 20 bis 50 mm sind besonders vorteilhaft.

In einer besonders bevorzugten Ausführungsform umfasst die Einlochdüse oder die Mehrlochdüse nicht rotationssymmetrische Öffnungen, insbesondere sternförmige oder ringförmige Querschnitte. Auch kreuzförmige oder geschlitzte Öffnungen können vorteilhaft sein. Von Bedeutung für derartige von der Kreisform abweichende Ausführungsformen ist, dass der hydraulische Durchmesser gegenüber einer rotationssymmetrischen Öffnung verringert wird. Unter dem hydraulischen Durchmesser versteht man das Vierfache der Querschnittsfläche der Öffnung dividiert durch den Umfang der Öffnung. Vorteilhaft werden hydraulische Durchmesser von 5 bis 100 mm, vorzugsweise von 10 bis 80 mm, besonders vorzugsweise 20 bis 50 mm jeder Öffnung verwendet.

Eine Ausgestaltung der Treibstrahldüse oder der Treibstrahldüsen als Mehrlochdüse ist besonders bevorzugt.

Jede Treibstrahldüse ist in vertikaler Richtung oberhalb einer Mischkammer angeordnet. In einer bevorzugten Ausführungsform ist jeder Treibstrahldüse eine eigene Mischkammer zugeordnet, wobei besonders bevorzugt Mehrlochdüsen verwendet werden.

In der Mischkammer findet im Betrieb der vorliegenden Erfindung ein Impulsaustausch statt.

Die Treibstrahldüse ist vorzugsweise so angebracht, dass im Betrieb das untere Ende der Düse um das 0 bis 10-fache des Durchmessers der Treibstrahldüse, vorzugsweise das 0,5 bis 2 fache, von der Oberfläche des Reaktionsmediums beabstandet ist. Hierdurch wird eine optimale Ansaugwirkung in Bezug auf den gasförmigen Reaktanden erzielt.

In einer alternativen Ausführungsform taucht die Treibstrahldüse zu einem gewissen Anteil ihrer Baulänge durch den Gas-Flüssig- Phasentrennspiegel in die Flüssigkeit ein. Der Gaseintrag erfolgt dann nur noch über die Frischgaszuführung unterhalb des Wärmeübertragers.

Um die Durchmischung zwischen den Reaktanden und dem Reaktionsmedium weiter zu verbessern, kann pro Treibstrahldüse mindestens ein Einlass für den flüssigen Reaktanden vorgesehen werden.

Die erfindungsgemäße Vorrichtung umfasst zudem mindestens einen Auslass zur Entnahme des Reaktionsmediums aus dem Reaktor. Der Auslass zur Entnahme des Reaktionsmediums aus dem Reaktor ist vorzugsweise am unteren Ende des Reaktors angebracht. In einer bevorzugten Ausführungsform befindet sich der Auslass unterhalb des Mittels zur Umlenkung der internen Zirkulationsströmung. Demzufolge befindet sich der Auslass vorzugsweise außerhalb der internen Zirkulationsströmung im Betrieb. Die Entnahme des Reaktionsmediums aus der internen Zirkulationsströmung erfolgt durch ein weiteres Mittel, das von dem Auslass zur Entnahme aus dem Reaktor zu unterscheiden ist.

Von Bedeutung ist hier die Art und Weise der Entnahme des Reaktionsmediums aus der internen Zirkulationsströmung, bevor das Reaktionsmedium dem Reaktor entnommen wird. Die Entnahme des Reaktionsmediums aus der internen Zirkulationsströmung wird vorzugsweise unmittelbar vor dem Einlass für den flüssigen Reaktanden vorgenommen. Hierdurch wird erreicht, dass das Reaktionsprodukt der internen Zirkulationsströmung an einer Stelle entnommen wird, bei der maximaler Umsatz in Bezug auf den flüssigen Reaktanden vorliegt.

Vorzugsweise erfolgt die Entnahme des Reaktionsmediums aus der internen Zirkulationsströmung somit in unmittelbarer räumlicher Nähe zum Einlass zur Zuführung des flüssigen Reaktanden.

Es ist somit bevorzugt, wenn das Reaktionsmedium, nachdem es der internen Zirkulationsströmung entnommen wurde, in einer Schicht entlang der Außenwand des Reaktors nach unten strömt, bevor es aus dem Reaktor entnommen wird. Besonders bevorzugt strömt das Reaktionsmedium hierbei durch das Innere von im äußeren Bereich des Reaktionsraumes angeordneten Wärmeübertragerrohren nach unten und wird somit auf dieser Strecke weiterhin gekühlt.

In einer alternativen Ausführungsform befindet sich der Auslass zur Entnahme des Reaktionsmediums aus dem Reaktor im oberen Bereich des Reaktors. In dieser Ausführungsform befindet sich der Auslass zur Entnahme des Reaktionsmediums aus dem Reaktor in räumlicher Nähe zur Entnahme des Reaktionsmediums aus der internen Zirkulationsströmung.

Der Auslass zur Entnahme des Reaktionsmediums aus dem Reaktor und die mindestens eine Treibstrahldüse zum Eintrag des Reaktionsmediums stehen im Betrieb der erfindungsgemäßen Vorrichtung über einen externen Kreislauf miteinander in Fluidverbindung.

In einer weiteren alternativen Ausführungsform erfolgt die Entnahme des Reaktionsmediums aus der internen Zirkulationsströmung im unteren Bereich des Reaktors und strömt anschließend an dem Mittel zur Umlenkung der internen Zirkulationsströmung vorbei nach unten, wo es dem Reaktor entnommen wird und der externen Zirkulationsströmung zugeführt wird (d. h. durch den Pumpenstrom angesaugt wird). Diese Ausführungsform ist jedoch weniger bevorzugt.

Im Betrieb der erfindungsgemäßen Vorrichtung ist mindestens eine Pumpe in dem externen Kreislauf angeordnet, der die Fluidverbindung zwischen dem Auslass zur Entnahme des Reaktionsmediums aus dem Reaktor und der mindestens einen Treibstrahldüse bildet, so dass das Reaktionsmedium entsprechend vom Auslass zur Entnahme aus dem Reaktor zur Treibstrahldüse gepumpt wird. Die erfindungsgemäße Vorrichtung umfasst deshalb mindestens eine Pumpe, die in einem externen Kreislauf angeordnet ist und den Auslass zur Entnahme des Reaktionsmediums aus dem Reaktor mit der mindestens einen Treibstrahldüse fluidisch verbindet. Somit erfolgt der Energieeintrag für die externe Zirkulationsströmung durch mindestens eine Pumpe, weshalb man hier von einem Pumpenstrom sprechen kann. Die interne Zirkulationsströmung wird durch die Treibstrahldüse angetrieben, weshalb man hier von einem Ansaugstrom sprechen kann.

Jede Mischkammer bildet mit der oberhalb angeordneten Treibstrahldüse oder Treibstrahldüsen eine Strahlpumpe, die als Ejektor bezeichnet wird. Der Ejektor umfasst in einer bevorzugten Ausführungsform darüber hinaus einen Diffusor, welcher mit dem unteren Ende der Mischkammer verbunden ist. Der Diffusor wird somit im Rahmen der vorliegenden Erfindung von der Mischkammer unterschieden. Vorzugsweise wird die Mischkammer durch ein zylindrisches Einsteckrohr gebildet.

Ein Diffusor wird durch eine Erweiterung, vorzugsweise eine trichterförmige Erweiterung, des Querschnitts gegenüber der Mischkammer gebildet. Insbesondere ist die mindestens eine Mischkammer am unteren Ende jeweils mit einem Diffusor verbunden. Darunter ist insbesondere zu verstehen, dass Diffusor und Mischkammer miteinander physisch verbunden sind, d.h. unmittelbar aneinander angrenzen und somit eine Einheit bilden.

Bei Verwendung eines Diffusors tritt eine Verbesserung des Stoffübergangs an der Phasengrenzfläche ein, indem die kinetische Energie eines Flüssigkeitsstrahls hoher Geschwindigkeit zum Ansaugen und Dispergieren der Gasphase genutzt wird. Durch den hohen Energieeintrag wird im Ejektor eine hohe Turbulenz und große Scherkräfte erzeugt mit der Folge, dass das Gas in Form sehr kleiner Bläschen dispergiert wird, d. h. dass eine sehr hohe volumenspezifische Gas-Flüssigkeits-Grenzfläche erzeugt wird. Die Funktion des Diffusors besteht insgesamt in der Erhöhung des Wirkungsgrades der internen Zirkulationsströmung.

In vertikaler Richtung unterhalb des Diffusors befindet sich in einer bevorzugten Ausführungsform eine zylindrische Verlängerung des Diffusors in Form eines Rohres mit konstantem Durchmesser.

In einer besonders bevorzugten Ausführungsform umfasst entsprechend ein in Treibstrahlrichtung von oben nach unten angeordneter Ejektor, der durch ein oder mehrere Treibstrahldüsen, eine Mischkammer, ggf. Diffusor und ggf. weitere zylinderförmige Abschnitte gebildet wird, unterhalb der Treibstrahldüsen mindestens 3 Abschnitte: einen oberen Abschnitt, der einen im Wesentlichen runden Querschnitt mit dem Durchmesser d1 aufweist, einen Diffusor als mittleren Abschnitt, dessen Querschnitt sich gegenüber dem oberen Abschnitt von oben nach unten erweitert, und einen unteren Abschnitt, der einen im Wesentlichen runden Querschnitt mit dem Durchmesser d2 aufweist, wobei d2 > d1.

In einer bevorzugten Ausführungsform weist das Einsteckrohr als Mischkammer am oberen Ende einen gerundeten Einlauf auf, d. h. das Einsteckrohr weist im Bereich des oberen Endes eine geringfügige Aufweitung des Innendurchmessers auf. Dies führt zu einer verbesserten Ansaugwirkung in Bezug auf das Reaktionsmedium unter Vermeidung von Strömungsverlusten.

In einer bevorzugten Ausführungsform werden mehrere Ejektoren verwendet. Hierdurch wird das Einmischen der Eduktströme beschleunigt. Darüber hinaus ist diese Ausführungsform besonders effizient in Kombination mit der weiter unten ausgeführten Querstromfiltration, da diese zu einer Vorverteilung von Strömen führt. Die einzelnen Rohre dieser Vorverteilung können so vorteilhaft mit jeweils einer Treibstrahldüse verbunden werden.

Grundsätzlich beträgt die für eine ausreichende Mischung erforderliche Mischlänge in der Regel das Fünf-bis Zehnfache des Durchmessers der Mischkammer. Somit erhöht sich bei mehreren Ejektoren insgesamt die Effizienz der Vermischung, d. h., die ausreichende Durchmischung stellt sich in der Strömung schneller ein. Auch der direkte Gaseintrag durch die Treibstrahldüse wird so verbessert, indem die Kontaktfläche zwischen Gas und Flüssigkeit erhöht wird.

In einer weiteren Ausführungsform ist die Treibstrahldüse, die im oberen Bereich des Reaktors angeordnet ist, als Ringspaltdüse ausgebildet, und der gasförmige Reaktand, vorzugsweise Wasserstoff, wird über einen Ringspalt an der Außenwand der als Zweistrahldüse ausgebildeten Treibstrahldüse in den Reaktor geleitet.

Um im Betrieb des Reaktors eine interne Zirkulationsströmung einzustellen, sind Mittel vorgesehen, die den Strom unterhalb des Wärmeübertragers seitlich umlenken, wodurch sich im Reaktor eine interne Zirkulationsströmung ergibt.

In einer Ausführungsform der Erfindung ist in dem externen Kreislauf, der auch als externe Schlaufenströmung bezeichnet werden kann, ein Wärmeübertrager vorgesehen. Ein etwaiger restlicher Teil der Reaktionswärme kann somit über einen Wärmeübertrager abgeführt werden, der in dem externen Kreislauf angeordnet ist. Bevorzugt kommt dabei ein Rohrbündel-Wärmeübertrager zum Einsatz.

Der Gasraum kann auch als Gasabscheideraum bezeichnet werden. In einer bevorzugten Ausführungsform befinden sich oberhalb des Wärmeübertragers unterhalb des Flüssigkeitsspiegels Mittel zur Gasabscheidung, vorzugsweise Bleche, die sich in horizontaler Ebene nicht bis an den Rand des Reaktors erstrecken, damit im äußeren Bereich das Reaktionsmedium ungestört zum Auslass aus dem Reaktor fließen kann. In der Mitte weisen die Mittel zur Gasabscheidung mindestens eine Aussparung auf, damit im Bereich der Treibstrahldüsen Gas aufsteigen kann. An den Blechen sammelt sich das Gas, welches im Reaktionsraum nach oben steigt, und wird durch die interne Zirkulationsströmung (Ansaugstrom) teilweise wieder nach unten gesaugt.

Das Volumen des Gases im Reaktionsmedium sollte im Betrieb entsprechend der Stoffeigenschaften auf 5 bis 20 Vol.-% bezogen auf das Volumen des Reaktionsmediums eingestellt werden.

Wird der Stand des Flüssigkeitsspiegels im Reaktor (z.B. durch Verwendung einer Mehrlochdüse) konstant gehalten, wird der Gasgehalt bevorzugt durch Anpassung der ausgeschleusten Menge an Reaktionsprodukt geregelt. Alternativ kann auch der Stand des Flüssigkeitsspiegels im Reaktor durch Anpassung der ausgeschleusten Menge an Reaktionsprodukt geregelt werden, dann kann der Gasgehalt z.B. durch die Menge des unterhalb des Wärmeübertrager dosierten Frischgases beeinflusst werden.

Der Reaktor kann prinzipiell aus jedem Material gefertigt werden, das über die notwendige mechanische und thermische Belastbarkeit und Produktverträglichkeit verfügt. Bevorzugt wird der Reaktor aus Stahl (z.B. 1.0037 oder 1.0565), besonders bevorzugt aus Edelstahl (z.B. 1.4541 oder 1.4571) und/oder aus Duplex-Stahl (z.B. 1.4462) gefertigt. Möglich sind selbstverständlich auch Kombinationen bei denen z.B. Produkt berührende Teile aus Edelstahl und/oder Duplex und nicht Produkt berührende Teile aus unlegiertem Stahl gefertigt werden.

### Verfahren

Die erfindungsgemäße Vorrichtung wird vorzugsweise für die Umsetzung von Gasen mit Flüssigkeiten eingesetzt. Vorzugsweise ist die Umsetzung eine Hydrierung, Oxidation oder Acetylierung, besonders bevorzugt eine Hydrierung. Dabei werden vorzugsweise nitroaromatische Verbindungen hydriert.

Vorzugsweise werden dabei im Rahmen des erfindungsgemäßen Verfahrens aromatische Nitroverbindungen mit einer oder mehreren Nitrogruppen und 6 bis 18 C-Atomen, bsp. Nitrobenzole, wie z. B. Nitrobenzol, 1,3-Dinitrobenzol, Nitrotoluole, wie z. B. 2,4-, 2,6-Dinitrotoluol, 2,4,6-Trinitrotoluol, Nitroxylole, wie z. B. 1,2-Dimethyl-3-, 1,2 Dimethyl-4-, 1,4-Dimethyl-2-, 1,3-Dimethyl-2-, 2,4-Dimethyl-1- und 1, 3-Dimethyl-5-nitrobenzol, Nitronaphthaline, wie z. B. 1-, 2-Nitronaphthalin, 1,5 und 1,8-Dinitronaphthalin, Chlornitrobenzole, wie z. B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z. B. 4-Chlor-2, 4-Chlor-3-, 2-Chlor-4-und 2-Chlor-6-nitrotoluol, Nitroaniline, wie z. B. o-, m-, p-Nitroanilin ; Nitroalkohole, wie z. B. Tris (hydroxymethyl) nitromethan, 2-Nitro-2-methyl-, 2-Nitro-2-ethyl-1, 3-propandiol, 2-Nitro-I-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Mononitrotoluol oder Dinitrotoluol, und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol sowie Anteilen von 1 bis 4 Prozent an vicinalem DNT und 0,5 bis 1,5 % an 2,5- und 3,5-Dinitrotoluol aufweisen, zu den entsprechenden Aminen hydriert. Besonders bevorzugt wird das technische DNT-Gemisch in der Isomerenzusammensetzung eingesetzt, in der es bei der zweistufigen Nitrierung von Toluol anfällt.

Insbesondere bei der Hydrierung von Dinitrotoluolisomeren zu den entsprechenden Toluylendiaminderivaten (TDA), bei der Hydrierung von o- oder p-Mononitrotoluol zu o- bzw. p-Toluidin und bei der Hydrierung von Mononitrobenzol zu Anilin kann das erfindungsgemäße Verfahren vorteilhaft eingesetzt werden.

Nachfolgend wir die bevorzugte Ausführungsform der Hydrierung von Dinitrotoluol näher erläutert.

Dinitrotoluol wird vorzugsweise am oberen Ende des Reaktors zugeführt, bevorzugt im Gasraum oberhalb des Flüssigkeitsspiegels im Reaktor. Vorteilhafterweise wird das Dinitrotoluol zwecks gleichmäßiger Zuführung direkt in die Mischkammer dosiert, besonders bevorzugt in die vom Strahl aus der Treibstrahldüse erzeugten Trombe. Dabei wird vorzugsweise eine Mono- und/oder Polynitroverbindung in reiner Form, als Mischung mit dem entsprechenden Mono- und/oder Polyamin, als Mischung mit dem entsprechenden Mono-und/oder Polyamin und Wasser oder als Mischung mit dem entsprechenden Mono-und/oder Polyamin, Wasser und einem insbesondere alkoholischen Lösungsmittel eingesetzt. Die aromatische Mono-und/oder Polynitroverbindung wird fein verteilt in das Gemisch eingetragen.

In einer bevorzugten Ausführungsform des erfindungsgemäßen Verfahrens wird die Zuführung des Nitroaromaten durch eine Zulaufleitung sowie eine Dosiereinrichtung ohne geometrische Möglichkeit einer Totraumbildung realisiert. Entsprechende Einheiten bzw. Vorrichtungen sind dem Fachmann an sich bekannt. Von Vorteil ist es dabei, dass bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das aromatische Amin, d. h. das entsprechende Reaktionsprodukt, Wasser und Katalysator, im Reaktor und/oder im Umpumpkreis erfolgen kann. Dies wird insbesondere dadurch realisiert, dass der Abstand zwischen der Zuführung des Nitroaromaten und der flüssigen Phase beispielsweise 0,01 bis 3 m, bevorzugt 0,05 bis 1,0 m beträgt.

In einer weiter bevorzugten Ausführungsform des erfindungsgemäßen Verfahrens wird die Zuführung des Nitroaromaten über eine oder mehrere unabhängige Rohrleitungen realisiert, deren teilweise oder vollständige Verblockung messtechnisch erfasst werden kann.

Entsprechende Rohrleitungen sind dem Fachmann an sich bekannt. Die bevorzugt eingesetzten Rohrleitungen sind beispielsweise aus Metall, beispielsweise Schwarzstahl oder Edelstahl, gefertigt. Die Rohrleitungen weisen bevorzugt Durchmesser auf, die sich nach den Mengen an Nitroaromaten, welche zugeführt werden, bemessen.

Im Allgemeinen können die bevorzugt eingesetzten Rohrleitungen jeden geeigneten Querschnitt aufweisen. Bevorzugt werden Rohrleitungen mit DN50 oder kleiner, besonders bevorzugt DN40 oder DN25 verwendet, da diese als Detonationssperre wirken.

In einer bevorzugten Ausführungsform weist der Austrittsquerschnitt der Zuführung des Nitroaromaten an den Enden der einen oder mehreren unabhängigen Rohrleitung(en) eine Verengung oder eine von der rotationssymmetrischen Form abweichende Gestalt auf. Der austretende Nitroaromat-Strahl kann laminares oder turbulentes Verhalten aufweisen. Eine entsprechende Realisierung wird beispielsweise in der WO2012/123469 beschrieben.

Durch die Einleitung des Dinitrotoluols in die Gasphase oberhalb des Flüssigkeitsspiegels wird verhindert, dass Reaktionsprodukt zurück in die Zuführleitung für Dinitrotoluol strömt und dass es dadurch zur Zersetzung oder Explosion des Dinitrotoluols führt. Reines Dinitrotoluol hat eine Zersetzungstemperatur von etwa 260°C, die Zersetzungstemperatur sinkt jedoch drastisch sobald Toluylendiamin und Katalysator zugemischt wird, bis auf 100°C.

Daher ist es auch vorteilhaft, die Zuführleitung des Dinitrotoluols bei Unterbrechungen der Produktion oder Abstellungen der Anlage mit heißem Wasser frei zu spülen.

Vorzugsweise wird die Konzentration an Nitrogruppen, d. h. die Summe der Produkte aller enthaltener Nitroaromaten, multipliziert jeweils mit der Anzahl ihrer Nitrogruppen pro Molekül, beispielsweise für die Hydrierung von Dinitrotoluol (DNT) c(Nitroaromaten) = c(DNT) · 2 + c(ANT) · 1 (ANT = Amino-nitro-toluol), oder für die Hydrierung von ortho-Nitrotoluol (o-NT) c(Nitroaromaten) = c(o-NT) · 1, in der Flüssigphase des Produktaustrages aus dem Reaktor im Bereich zwischen dem Reaktor und der nachgeschalteten Produktabtrenneinheit auf einen Wert im Bereich von 0 bis 2000 Gew.-ppm, bevorzugt 0,5 bis 1000 Gew.-ppm, besonders bevorzugt 1 bis 200 Gew.-ppm und ganz besonders bevorzugt 1 bis 50 Gew.-ppm, bezogen auf das Gesamtgewicht der Flüssigphase des Produktaustrages aus dem Reaktor, eingestellt. Hierdurch kann sowohl die Nebenproduktbildung verringert als auch die Katalysatordesaktivierung weitgehend vermieden werden. Entsprechende Verfahren werden in der WO2011/144481 beschrieben. Die genannten Konzentrationsbereiche können hierbei umso leichter erreicht werden, je schneller die Einmischung des flüssigen Reaktanden (z.B. mittels einer hocheffizienten Treibstrahldüse mit Diffusor) erfolgt und je besser Kurzschlussströme unterbunden werden. Im Vergleich zu Reaktoren ohne derartige Hilfsmittel lassen sich diese Konzentrationen daher mit weniger Katalysator, höherer Raumzeitausbeute oder geringerer Temperatur (und damit häufig höherer Selektivität) erzielen.
Es ist vorteilhaft, durch geeignete Maßnahmen sicherzustellen, dass die Konzentration des Wasserstoffs im Reaktionsgemisch, das aus der internen Zirkulationsströmung in die externe Schlaufe strömt, 1 Vol.-%, bevorzugt 3 Vol.-%, bezogen auf das Gesamtvolumen des Reaktionsgemisches, das in der externen Schlaufe strömt, nicht unterschreitet. Hierzu ist es möglich, den Durchmesser des Reaktors oder die Abströmgeschwindigkeit des Reaktionsgemisches aus dem Reaktor oder die ggf. vorhandenen Gasabscheidungsbleche oberhalb des Wärmtauschers entsprechend auszulegen.

In einer weiteren Ausführungsform ist es möglich, die Mindestkonzentration des Wasserstoffs, der aus der internen Zirkulationsströmung in die externe Schlaufe strömt sicherzustellen, indem in das Reaktionsgemisch, das aus der internen Zirkulationsströmung in die externe Schlaufe strömt, möglichst nahe am Reaktor Wasserstoff zugeführt wird.

Das Reaktionsprodukt wird bevorzugt dem externen Kreislauf entnommen. Um den externen Wälzkreis antreiben zu können, muss eine Pumpe installiert werden, die neben Flüssigkeit auch Gas und suspendierten Feststoff fördern kann. Dabei sollten bis zu 20 Vol.-% Gas und 20 Gew.-% suspendierter Feststoff pumpbar sein. Zum Vermeiden von Ni-Ablagerungen bei Nihaltigen Katalysatoren oder zur Vermeidung einer zu starken Zerkleinerung von geträgerten Katalysatoren kann eine Anordnung von mehreren (bevorzugt zwei) Pumpen in Reihe oder die Verwendung einer mehrstufigen Pumpe sinnvoll sein.

Das Kühlmedium ist vorzugsweise Wasser. In einer bevorzugten Ausführungsform wird Wasser flüssig zugeführt und als Dampf entnommen. Der Dampf kann innerhalb jedes chemischen Anlagenkomplexes zur Wärme- oder Energiezufuhr verwertet werden. Darüber hinaus kann der Dampf auch in ein vorhandenes Dampfnetz am Standort der Anlage eingespeist werden.

Aus der freiwerdenden Reaktionswärme kann Dampf in sowohl in dem internen als auch einem ggf. vorhandenen externen Wärmeübertrager auf zwei Arten erzeugt werden: 1) Durch Verdampfung eines Teils des Kühlwassers in den Kühlrohren (Direktdampferzeugung) oder 2) durch Erhitzen des Kühlwassers auf einen Druck, der oberhalb des Druckes des zu erzeugenden Dampfes liegt, und anschließende Entspannung auf das Druckniveau des zu erzeugenden Dampfes (Entspannungsverdampfung). Bei dieser Entspannung verdampft ein Teil des Kühlwassers, und das Dampf/Wasser-Gemisch kühlt sich auf die dem Druck des Dampfes entsprechende Siedetemperatur ab.

Beide Arten der Verdampfung können sowohl im internen und als auch einem externen Wärmeübertrager angewendet werden. Ebenso ist eine Kombination beider Verdampfungsarten möglich, das heißt Direktverdampfung im internen Wärmeübertrager und Entspannungsverdampfung in einem externen Wärmeübertrager oder umgekehrt.

In einer alternativen Ausführungsform wird zumindest teilweise Wasser aus einem Sekundärkühlkreislauf als Kühlmedium genutzt. In einer bevorzugten alternativen Ausführungsform wird im internen Wärmeübertrager auf eine der beiden beschriebenen Arten Dampf erzeugt und ein externer Wärmeübertrager mit Wasser aus einem Sekundärkühlwasserkreislauf gekühlt.

Vorzugsweise erfolgt die Umsetzung im erfindungsgemäßen Verfahren in Gegenwart eines suspendierten oder gelösten besonders bevorzugt in Gegenwart eines suspendierten Katalysators.

Für die Hydrierung von Dinitrotoluol zu Toluylendiamin wurden eine Vielzahl von Katalysatoren entwickelt, wobei die Verbesserung von Ausbeute und Selektivität der Umsetzung sowie die Stabilität der Katalysatoren auch bei höheren Reaktionstemperaturen vorrangige Aufgaben bei der Entwicklung neuer Katalysatoren waren.

Gemäß einer ersten allgemeinen Ausführungsform der Erfindung wird das erfindungsgemäße Verfahren, wie oben ausgeführt, in Gegenwart eines Katalysators, insbesondere in Gegenwart eines Trägerkatalysators, der als Aktivkomponente Nickel allein oder zusammen mit mindestens einem Metall der I., V., VI. und/oder VIII. Nebengruppe des Periodensystems aufweist, durchgeführt. Die bevorzugt verwendeten Katalysatoren können technisch durch Aufbringen von Nickel und gegebenenfalls mindestens einem der oben genannten zusätzlichen Metalle auf einen geeigneten Träger hergestellt werden.

Vorzugsweise werden als Metalle der I., II., V., VI. und/oder VIII. Nebengruppe des Periodensystems Palladium, Platin, Rhodium, Eisen, Kobalt, Zink, Chrom, Vanadium, Kupfer, Silber oder ein Gemisch aus zwei oder mehreren davon verwendet.

Als besonders geeignet haben sich Katalysatoren erwiesen, die als Aktivmasse ein oder mehrere Metalle, ausgewählt aus der Gruppe Platin, Palladium, Rhodium und Ruthenium und daneben ein oder mehrere weitere Metalle, ausgewählt aus der Gruppe Nickel, Kobalt, Eisen und Zink enthalten, und die auf einem inerten Träger aufgebracht ist.

Gemäß einer bevorzugten Ausführungsform der Erfindung weist der Katalysator einen Nickelgehalt von 0,1 bis 99 Gew.-%, bevorzugt von 1 bis 90 Gew.-%, besonders bevorzugt von 25 bis 85 Gew.-% und ganz besonders bevorzugt von 60 bis 80 Gew.-%, bezogen auf das Gesamtgewicht des Katalysators auf.

Gemäß einer bevorzugten Ausführungsform der Erfindung enthält der Katalysator Ni und Platin. Gemäß einer weiteren bevorzugten Ausführungsform der Erfindung enthält der Katalysator Ni und AI; gemäß einer weiteren besonders bevorzugten Ausführungsform enthält der Katalysator Nickel, Palladium und Eisen.

Besonders vorteilhaft sind Hydrierkatalysatoren, die Platin und Nickel auf einem Träger in Form einer Legierung mit einem Atomverhältnis von Nickel zu Platin, bestimmt mittels EDXS (Energy Dispersive X-Ray Spectroscopy), in der Legierung von 30 : 70 bis 70 : 30, enthalten. Das Atomverhältnis von Nickel zu Platin, bestimmt mittels EDXS (Energy Dispersive X-Ray Spectroscopy), beträgt insbesondere von 45 : 55 bis 55 : 45.

Als Trägermaterialien werden vorzugsweise Aktivkohle, Ruß, Graphit, oder oxidische Trägerkomponenten wie Siliziumdioxid, Siliziumcarbid, Kieselgur, Aluminiumoxid, Magnesiumoxid, Titandioxid, Zirkoniumdioxid und/oder Hafniumdioxid oder ein Gemisch aus zwei oder mehr davon, besonders bevorzugt Zirkoniumdioxid, ZrO₂, HfO₂ und/oder SiO₂, ZrO₂ und/oder SiO₂, ZrO₂, HfO₂, verwendet.

Die verwendeten Träger sind vorzugsweise mesoporös und besitzen einen mittleren Porendurchmesser von 35 bis 50 nm und eine spezifische Oberfläche von 50 bis 250 m²/g. Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgt durch Hg-Porosimetrie, insbesondere nach DIN 66133.

Das Aufbringen von Nickel und gegebenenfalls des mindestens einen weiteren Metalls kann durch die üblichen geeigneten Verfahren, die dem Fachmann auf dem Gebiet der Katalysatortechnik bekannt sind, erreicht werden. Die mit dem Metall bzw. Metallsalz beschichteten, durch gemeinsame Fällung beschichteten oder getränkten Träger werden anschließend nach bekannten Verfahren getrocknet und kalziniert. Darauf folgend werden die beschichteten Träger durch Behandlung derselben in einem Gasstrom, der freien Wasserstoff enthält, aktiviert. Diese Aktivierung findet zumeist bei Temperaturen im Bereich von 30 bis 600 °C, vorzugsweise im Bereich von 80 bis 150 °C und insbesondere bevorzugt bei 100 °C statt. Der Gasstrom besteht vorzugsweise aus 50 bis 100 Vol.-% Wasserstoff und 0 bis 50 Vol.-% Stickstoff. Der für den bevorzugten Einsatz hergestellte Katalysator weist nach einstündiger Reduktion bei 100 °C einen Reduktionsgrad von mindestens 70 % auf.

Die so erhaltenen Trägerkatalysatoren besitzen im Allgemeinen eine Nickel-Metalloberfläche von ungefähr 10 bis ungefähr 50 m²/g, vorzugsweise ungefähr 20 bis ungefähr 60 m²/g. Der Nickelgehalt der im Rahmen des erfindungsgemäßen Verfahrens eingesetzten Katalysatoren liegt allgemein im Bereich von 0,1 bis 99 Gew.-%, bevorzugt im Bereich von 1 bis 90 Gew.-%, besonders bevorzugt im Bereich von 25 bis 85 Gew.-%, bezogen auf das Gesamtgewicht der eingesetzten Katalysatoren.

Geeignete Katalysatoren dieser Ausführungsform sind z.B. in den Druckschriften EP 1 161 297 A1 und EP 1 165 231 A1 beschrieben.

Gemäß einer zweiten Ausführungsform der Erfindung werden im Rahmen des erfindungsgemäßen Verfahrens aktivierte Nickelkatalysatoren, wie beispielsweise in der WO 2008/145179 A1 beschrieben, eingesetzt. Demgemäß werden gemäß einer bevorzugten Ausführungsform der Erfindung aktivierte Nickelkatalysatoren auf Basis einer Ni/Al-Legierung, die ein oder mehrere Metalle ausgewählt aus der Gruppe Mg, Ce, Ti, V, Nb, Cr, W, Mn, Re, Fe, Ru, Co, Rh, Ir, Pt, Cu, Ag, Au und Bi enthalten können, eingesetzt. Der Dotierungsgrad liegt dabei im Bereich von 0,05 Gew.-% bis 20 Gew.-% für jedes Dotierungselement. Die mittlere Partikelgröße der eingesetzten Katalysatoren ist < 25 µm.

Gemäß einer dritten Ausführungsform der Erfindung werden im Rahmen des erfindungsgemäßen Verfahrens Katalysatoren eingesetzt, die beispielsweise in der WO 2008/138784 A1 beschrieben sind. Gegenstand der Erfindung ist somit gemäß einer bevorzugten Ausführungsform der Erfindung weiterhin die Verwendung von Hydrierkatalysatoren, enthaltend als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger, zur Herstellung von aromatischen Aminen durch katalytische Hydrierung der entsprechenden Nitroverbindungen, insbesondere zur Herstellung von Toluylendiamin durch Hydrierung von Dinitrotoluol. Das zusätzliche Element ist vorzugsweise ausgewählt aus der Gruppe, enthaltend Kobalt, Eisen, Vanadium, Bismut und Zinn.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide wie z.B. ZrO₂, TiO₂, Al₂O₃ eingesetzt. Bei Graphit sind die HSAG (High Surface Area Graphite) mit einer Oberfläche von 50 bis 300 m²/g besonders bevorzugt.

### Besonders bevorzugt sind Aktivkohlen, insbesondere physikalisch oder chemisch aktivierte Aktivkohlen, oder Ruße, wie Acetylenruß

Die Herstellung der bevorzugten Katalysatoren erfolgt beispielsweise, indem der Träger vorgelegt und mit einer wässrigen Lösung der Palladium- und Nickelsalze zusammen mit dem Zusatzelement gebracht wird. Dabei sollte die Menge des zur Lösung der Salze verwendeten Wassers so bemessen sein, dass eine knetbare Paste entsteht. Vorzugsweise wird das Wasser in einer Menge von 100 bis 200 Gew.-% der Trägermasse eingesetzt. Als Metallsalze kommen insbesondere Nitrate oder Chloride zum Einsatz, wobei Nitrate wegen ihrer geringeren Korrosivität bevorzugt sind. Die Paste wird gemischt und danach das Wasser bei geringem Druck und Temperaturen im Bereich zwischen 50 und 100 °C verdampft, beispielsweise in einem Rotationsverdampfer oder einem Ofen. Aus Sicherheitsgründen kann das Verdampfen in einem Stickstoffstrom erfolgen. Die Fixierung der Metalle auf dem Träger kann bei der Verwendung von Chloriden als Metallsalze durch Reduktion mit Wasserstoff erfolgen. Hierbei kann jedoch Korrosion auftreten. Bevorzugt werden die Metalle daher alkalisch fixiert. Dies erfolgt insbesondere durch Zugabe einer wässrigen Lösung von Alkalicarbonaten und nachfolgendes anionfrei waschen des Trägers. Alternativ können die Metalle auch alkalisch, insbesondere bei einem pH-Wert im Bereich von 8 bis 9, aus einer überstehenden Lösung auf den Träger gefällt werden. Danach wird der Träger getrocknet, vorzugsweise wie oben beschrieben, und mit Wasserstoff reduziert. Dies kann beispielsweise in einem Drehkugelofen geschehen. Vor dem Ausbau des Katalysators wird dieser passiviert, beispielsweise unter einem Inertgas, wie Stickstoff, der Spuren von Luft, vorzugsweise nicht mehr als 10 Vol.-%, enthält.

Die nach diesem Verfahren hergestellten bevorzugten Hydrierkatalysatoren enthalten vorzugsweise 0,5 bis 5 Gew.-% Palladium, 10 bis 20 Gew.-% Nickel und 0,5 bis 5 Gew.-% des Zusatzelementes.

Gemäß einer weiteren Ausführungsform der Herstellung der bevorzugt eingesetzten Hydrierkatalysatoren erfolgt die Reduktion der Katalysatoren durch Zusatz von reduzierend wirkenden Salzen, wie Ammoniumcarboxylaten oder Alkalicarboxylaten, beispielsweise Ammoniumformiat oder Natriumformiat. Dazu wird der Träger mit Wasser suspendiert und gleichzeitig oder nach der Suspendierung die Lösungen der Metallsalze zugegeben. Als Metallsalze kommen insbesondere Nitrate oder Chloride zum Einsatz, wobei Nitrate wegen ihrer geringeren Korrosivität bevorzugt sind. Zu dieser Lösung werden die reduzierend wirkenden Salze zugegeben und die Suspension erhitzt, beispielsweise durch Kochen unter Rückfluss. Anschließend werden die Katalysatoren anionenfrei gewaschen und filtriert, beispielsweise durch eine Filterpresse oder eine Zentrifuge, und als feuchte Paste verwendet. Weitere Beispiele für die Herstellung der bevorzugt eingesetzten Katalysatoren finden sich in der WO 2005/037768 A1.

Die Hydrierung des Dinitrotoluols, des Mononitrotoluols und des Mononitrobenzols kann in Lösung durchgeführt werden. Als Lösungsmittel werden die dafür üblichen Stoffe, insbesondere niedere Alkohole, vorzugsweise Ethanol oder Methanol, eingesetzt. Aufgrund der optimalen Strömungsverhältnisse und der sofortigen Abfuhr der Reaktionswärme in dem erfindungsgemäß verwendeten Reaktor ist es möglich, die Hydrierung auch ohne Lösungsmittel durchzuführen. Das hat die Vorteile, dass das Volumen der Reaktionsmischung geringer ist, was eine kleinere Dimensionierung des Reaktors sowie der Pumpen und Rohrleitungen ermöglicht, dass Nebenreaktionen zwischen dem Lösungsmittel und den Edukten ausgeschlossen werden sowie der Aufwand für die Aufarbeitung der Endprodukte verringert wird.

Der überwiegende Teil des Reaktionsgemisches wird in der internen Schlaufenströmung geführt, lediglich ein geringer Anteil des Reaktionsgemisches wird extern umgepumpt und sorgt so für den Antrieb der Schlaufenströmung. Das Verhältnis der Volumenströme von interner Schlaufenströmung zu externer Schlaufenströmung beträgt 1 : 1 bis 15 : 1, bevorzugt 3 : 1 bis 10 : 1. Durch den geringen Anteil der externen Schlaufenströmung an der gesamten Reaktionsmischung werden deutlich geringere Mengen an Katalysator pro Zeiteinheit über die Kreislaufpumpe umgewälzt als im Fall der Kühlung ausschließlich über einen externen Wärmeübertrager. Dies führt zu einer Verringerung der mechanischen Katalysatorbeanspruchung und damit zu einer längeren Lebensdauer des Katalysators. Außerdem gewährleistet diese Ausführungsform in Verbindung mit den integrierten Wärmeübertragern eine hohe Isothermie der Reaktion, d. h. einen sehr kleinen Temperaturgradienten über die Reaktorhöhe (typischerweise zwischen 1 und 10 K), da die Hydrierung praktisch vollständig in der internen Schlaufenströmung abläuft und die Reaktionswärme so bereits am Ort ihrer Entstehung abgeführt wird. Dabei werden Beschränkungen der Reaktionsgeschwindigkeit durch Stoff-und Wärmetransport praktisch vollständig ausgeschaltet. Nebenreaktionen, die durch Temperaturgradienten im Reaktionssystem begünstigt werden, werden praktisch vollständig unterdrückt. Die Reaktionsgeschwindigkeit ist dann nur noch durch die Reaktionskinetik begrenzt. Außerdem ist die Sicherheit des Verfahrens gegenüber einer Kühlung im äußeren Kreislauf verbessert, da die Reaktorkühlung auch dann noch funktioniert, wenn die Pumpe für den äußeren Kreislauf ausfällt.

Bevorzugt wird der Volumenstrom der internen Schlaufenströmung durch eine Messung der Druckdifferenz zwischen dem Gasraum und dem zylindrischen Mischraum direkt überwacht. Bei einer unzureichenden Schlaufenströmung - und damit unzureichender Durchmischung im Reaktor - wird die Zufuhr des flüssigen Eduktes unterbrochen um eine Desaktivierung des Katalysators zu verhindern.

Durch die Dispergierung der einzelnen Reaktanden wird in Verbindung mit den übrigen Reaktionsparametern eine intensive Durchmischung aller Komponenten bei niedrigen Substratkonzentrationen, hohen Stoffübergangskoeffizienten und hohen volumenspezifischen Phasengrenzflächen erreicht. Die Anordnung der Kühlrohre im Reaktor parallel zu den Reaktorwänden hat eine weitgehende Gradientenfreiheit des Reaktorinhalts bezüglich der Reaktionstemperatur zur Folge. Durch Vermeidung örtlicher Überhitzungen werden Nebenreaktionen deutlich zurückgedrängt und Katalysatordesaktivierung weitgehend vermieden. Es werden damit selbst bei niedrigen Katalysatorkonzentrationen hohe Raum-Zeit-Ausbeuten bei hoher Selektivität erreicht.

Auf Grund der geringen Konzentration des Nitroaromaten in der Reaktionsmischung wird eine Katalysatordesaktivierung weitgehend vermieden und eine thermische Zersetzung des Nitroaromaten kann sicher verhindert werden.

Die Hydrierung des Dinitrotoluols wird vorzugsweise bei Temperaturen von 80 bis 200 °C, bevorzugt von 110 bis 190 °C, und Drücken im Bereich von 10 bis 50 bar, bevorzugt von 15 bis 35 bar, durchgeführt.

Vorzugsweise wird der Katalysator in dem Reaktionsmedium suspendiert. Um das Reaktionsprodukt frei von Katalysatorteilchen entnehmen zu können, wird das Reaktionsprodukt im externen Kreislauf vom Katalysator abgetrennt. Die Produkt- bzw. Katalysatorabtrenneinheit ist allgemein ein Filter (z.B. ein Membranfilter / Querstromfilter), ein statischer Dekanter (z.B. ein Schwerkraftabscheider bzw. Settler, häufig ein Lamellenklärer) oder ein dynamischer Dekanter (z.B. eine Zentrifuge oder ein Düsenseparator). Der Katalysator wird von dem Produkt abgetrennt und anschließend (in der Regel als eingedickte Suspension) in den Reaktor zurückgeführt. Bevorzugt erfolgt der Produktaustrag unter Rückhaltung des Katalysators. Das Amin kann danach nach den üblichen und bekannten Verfahren, beispielsweise durch Destillation oder Extraktion, gereinigt werden.

Die Abtrennung des Reaktionsprodukts vom Katalysator erfolgt vorzugsweise mittels Membranfiltration.

Vorteil der Membranfiltration ist es, eine schnelle, vollständige und schonende Rückhaltung der Katalysatoren zu ermöglichen.

Die Membranfiltration wird vorzugsweise bei einem Druck auf der Suspensionsseite von 5 bis 50 bar, vorzugsweise 20 bis 35 bar, einer Druckdifferenz zwischen der Suspensionsseite und der Permeatseite von von 0,3 bar bis 5 bar und einer Strömungsgeschwindigkeit auf der Suspensionsseite von 1 bis 6 m/s durchgeführt wird. Unter Suspensionsseite wird die Seite des Membranfilters verstanden, auf der sich die Katalysator enthaltende Mischung befindet, unter Permeatseite wird die Seite des Membranfilters verstanden, auf der sich die katalysatorfreie Mischung befindet.

Die Membranfiltration kann kontinuierlich oder diskontinuierlich durchgeführt werden.

Bei der kontinuierlichen Durchführung wird ständig zumindest ein Teilstrom des Reaktionsgemisches durch einen Membranfilter gefahren. Bei dieser Ausführungsform des erfindungsgemäßen Verfahrens ist es bevorzugt, den Membranfilter in den externen Kreislauf eines Umlaufreaktors anzuordnen. Diese Ausführungsform des erfindungsgemäßen Verfahrens ist bevorzugt.

Bei der diskontinuierlichen Durchführung der Filtration wird das ausgeschleuste Reaktionsgemisch durch eine zuschaltbare Reinigungsstufe, bestehend aus Membranfilter und einer eigenen Zirkulationspumpe, geleitet. Bei einer anderen Ausgestaltung der diskontinuierlichen Filtration wird im Anschluss an die Reaktion das Reaktionsgemisch über einen Membranfilter gefahren. Diese Ausführungsform ist weniger bevorzugt, da hier der abgetrennte Katalysator höher aufkonzentiert werden muss.

Die für das Verfahren eingesetzten Filtermembranen können beispielsweise aus Keramik (z.B. α-Al₂O₃) oder Edelstahl (z.B. 1.4404) bestehen und haben, abhängig von der Partikelgröße des eingesetzten Katalysators, vorzugsweise zahlengewichtete mittlere Porendurchmesser im Bereich zwischen 10 nm und 20 Mikrometer, insbesondere im Bereich zwischen 50 nm und 10 Mikrometer und vorzugsweise zwischen 100 nm und 1 Mikrometer.

Geeignete Ausführungsformen einer Membranfiltration, insbesondere Querstromfiltration, sind dem Fachmann bekannt und werden beispielsweise in der WO2010/125025 beschrieben, deren Inhalt hiermit in diese Anmeldung einbezogen wird.

Trotz optimaler Reaktionsführung ist eine moderate Katalysatordesaktivierung nicht völlig zu verhindern. Deshalb enthält die äußere Schlaufe bevorzugt einen Anschluss zur Zufuhr von bevorzugt in Wasser suspendiertem Frischkatalysator und einen weiteren Ausgang zur Ausschleusung von katalysatorhaltigem Reaktionsprodukt der mit einer Katalysatorausschleuseunit verbunden ist. Diese besteht wiederum bevorzugt aus einem Auffangbehälter mindestens einem Umpumpkreislauf enthaltend mindestens einen Membranfilter.

Die Erfindung wird anhand von Ausführungsbeispielen und Zeichnungen näher erläutert. Dabei werden bevorzugte Ausführungsform beschrieben, ohne die Erfindung darauf beschränken zu wollen.

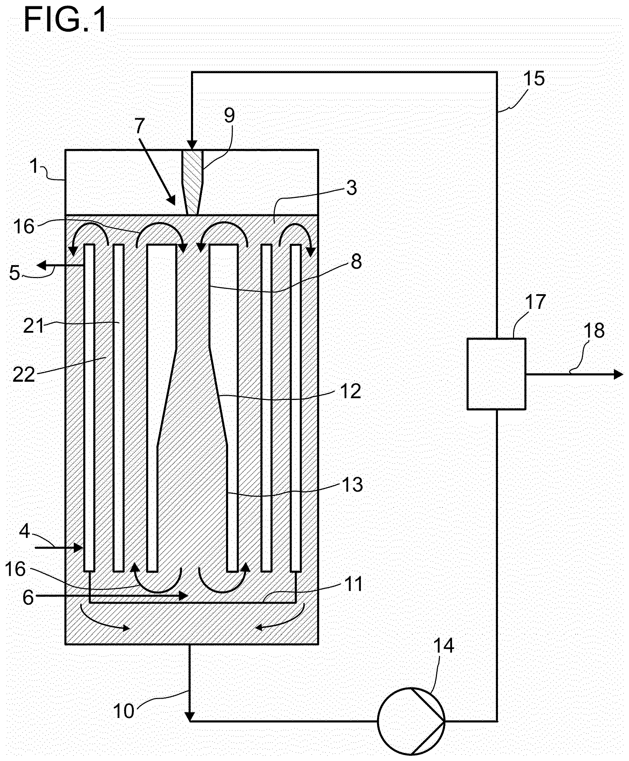

Die Abbildungen zeigen Vorrichtungen wobei die für das Reaktionsmedium vorgesehenen Bereiche schraffiert gekennzeichnet sind; die für das Kühlmedium vorgesehenen Bereiche sind nicht schraffiert. In den Figuren 1 bis 4 bedeuten im Einzelnen:
1 - vertikal ausgedehnter Reaktor
2 - Wärmeübertrager, der innerhalb des Reaktors angeordnet ist,
3 - Reaktionsraum
4 - Einlass zur Zuführung des Kühlmediums in den Wärmeübertrager,
5 - Auslass zur Entnahme des Kühlmediums aus dem Wärmeübertrager,
6 - Einlass zur Zuführung des gasförmigen Reaktanden in den Reaktionsraum,
7 - Einlass zur Zuführung des flüssigen Reaktanden in den Reaktionsraum,
8 - Mischkammer,
9 - nach unten gerichtete Treibstrahldüse zum Eintrag des Reaktionsmediums
10 - Auslass zur Entnahme des Reaktionsmediums aus dem Reaktor
11 - unterhalb des Wärmeübertragers (2) sowie unterhalb der Mischkammer (8) angeordnetes Mittel (11) zur Umlenkung des nach unten durch die Mischkammer (8) strömenden Reaktionsmediums
12 - Diffusor
13 - zylinderförmiger Abschnitt
14 - Pumpe
15 - externer Kreislauf
16 - interne Zirkulationsströmung
17 - Produktabtrenneinheit
18 - Produkt
20 - Wärmeübertragerrohre
21 - der die Wärmeübertragerrohre umgebende Bereich
22 - innere Bereich der Wärmeübertragerrohre

Figur 1 zeigt eine erfindungsgemäße Vorrichtung mit einem Ejektor (Schnitt in Längsrichtung).

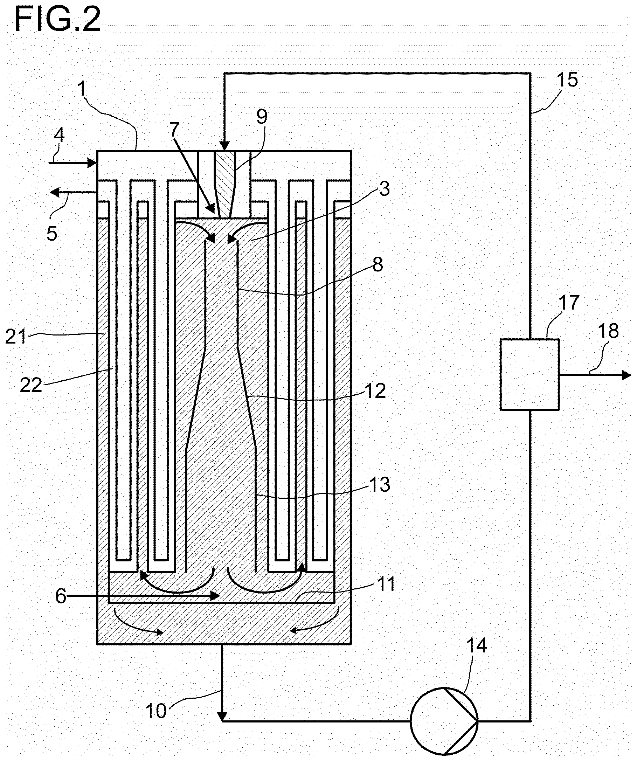

Figur 2 zeigt eine nicht erfindungsgemäße Vorrichtung mit einem Ejektor (Schnitt in Längsrichtung), wobei als Wärmetauscherrohre Fieldrohre verwendet werden.

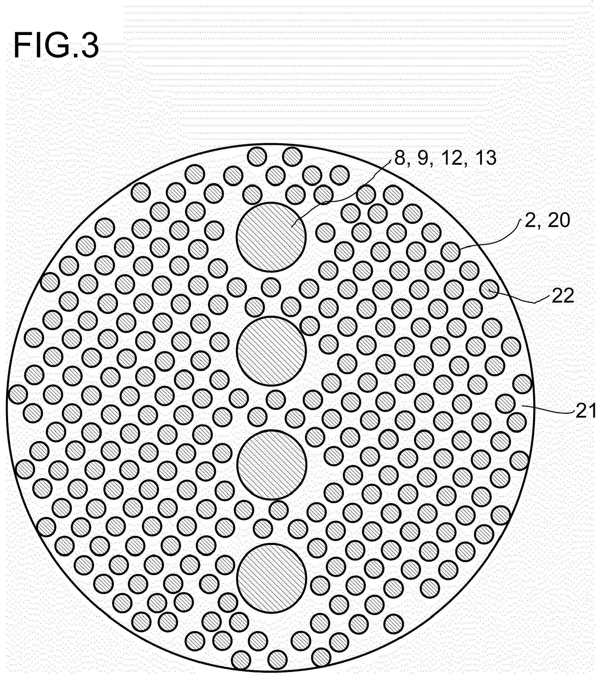

Figur 3 zeigt eine erfindungsgemäße Vorrichtung mit vier Ejektoren (Schnitt in Querrichtung). Der die Wärmeübertragerrohre umgebende Bereich (21) steht mit dem Einlass (4) zur Zuführung des Kühlmediums und dem Auslass (5) zur Entnahme des Kühlmediums in Fluidverbindung. Der innere Bereich der Wärmeübertragerrohre (22), der einen Teil des Reaktionsraums bildet, steht mit der mindestens einen Treibstrahldüse (9) und dem Auslass (10) zur Entnahme des Reaktionsmediums aus dem Reaktor in Fluidverbindung steht.

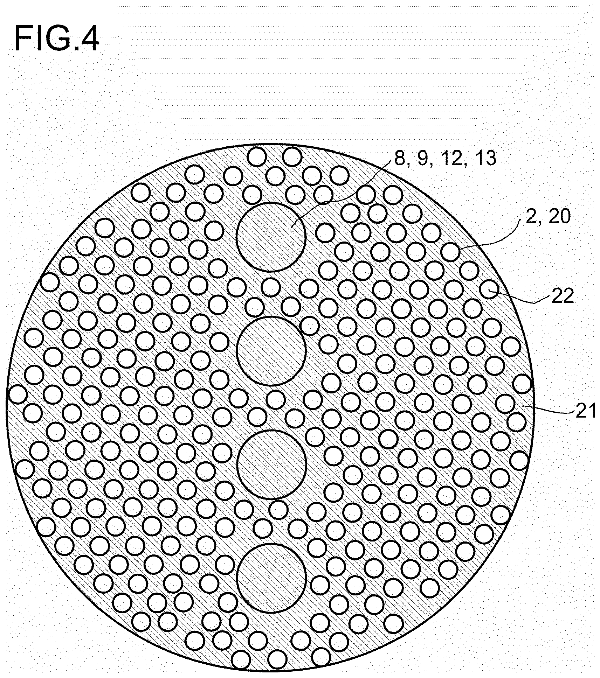

Figur 4 zeigt eine erfindungsgemäße Vorrichtung mit vier Ejektoren (Schnitt in Querrichtung). Der innere Bereich der Wärmeübertragerrohre (22) steht mit dem Einlass (4) zur Zuführung des Kühlmediums und dem Auslass (5) zur Entnahme des Kühlmediums in Fluidverbindung. Der die Wärmeübertragerrohre umgebende Bereich (21), der einen Teil des Reaktionsraums bildet, steht mit der mindestens einen Treibstrahldüse (9) und dem Auslass (10) zur Entnahme des Reaktionsmediums aus dem Reaktor in Fluidverbindung. Die Wärmeübertragerrohre (22) sind in dieser Ausführungsform typischerweise Fieldrohre.

### Beispiele

### Beispiel 1 (erfindungsgemäß)

In einem Reaktor gemäß Figur 1 und Figur 3 (ein Ejektor wie in Figur 1) mit einer Umlenkwanne als Mittel zur Umlenkung des nach unten durch die Mischkammer (8) strömenden Reaktionsmediums und mit einem Füllvolumen von 2,5 m³, einem Einsteckrohrvolumen von 0,5 m³, einem Volumen von 0,5 m³ unterhalb der Umlenkwanne, sowie mit einem externen Umpumpstrom von 0,1 m³/s und einem angesaugten Volumenstrom von 0,4 m³/s wurde die Eduktkonzentration in einer Simulation bei einer chemischen Reaktion 1. Ordnung mit einer Reaktionsgeschwindigkeitskonstante von 0,5 s⁻¹ am Reaktoraustritt auf 6% des ursprünglichen Mittelwertes am Einsteckrohreintritt abgebaut.

### Beispiel 2 (Vergleich)

Wie Beispiel 1, jedoch mit einer Prallplatte statt der Umlenkwanne, so dass direkt aus dem Einsteckrohr der externe Umpumpstrom in das Volumen unterhalb der Prallplatte geleitet wird, wurde die Eduktkonzentration bei der chemischen Reaktion 1. Ordnung am Reaktoraustritt auf 40% des ursprünglichen Wertes am Einsteckrohreintritt abgebaut.

## Patentansprüche

1. Vorrichtung zur kontinuierlichen Umsetzung von Flüssigkeiten mit Gasen, insbesondere zur Herstellung von Toluylendiamin durch Hydrierung von Dinitrotoluol, umfassend
- einen vertikal ausgedehnten Reaktor (1), in dem sich ein Reaktionsraum (3) befindet,
- mindestens einen Wärmeübertrager (2), der innerhalb des Reaktors (1) angeordnet ist,
- mindestens einen Einlass (4) zur Zuführung des Kühlmediums in den Wärmeübertrager (2),
- mindestens einen Auslass (5) zur Entnahme des Kühlmediums aus dem Wärmeübertrager (2),
- mindestens einen Einlass (6) zur Zuführung des gasförmigen Reaktanden in den Reaktionsraum (3),
- mindestens einen Einlass (7) zur Zuführung des flüssigen Reaktanden in den Reaktionsraum (3),
- mindestens eine Mischkammer (8),
- mindestens eine nach unten gerichtete Treibstrahldüse (9) zum Eintrag des Reaktionsmediums, deren Ausgang oberhalb der mindestens einen Mischkammer (8) angeordnet ist und die in Fluidverbindung mit dem Reaktionsraum (3) steht,
- mindestens einen Auslass (10) zur Entnahme des Reaktionsmediums aus dem Reaktor, und
- mindestens ein unterhalb des Wärmeübertragers (2) sowie unterhalb der Mischkammer (8) angeordnetes Mittel (11) zur Umlenkung des nach unten durch die Mischkammer (8) strömenden Reaktionsmediums dergestalt, dass das Reaktionsmedium durch den Wärmeübertrager (2) wieder nach oben strömt, und
- wobei ein externer Kreislauf (15) mit einer darin angeordneten Pumpe (14) eine Fluidverbindung zwischen dem Auslass (10) zur Entnahme des Reaktionsmediums aus dem Reaktor (1) und der mindestens einen Treibstrahldüse (9) zum Eintrag des Reaktionsmediums in den Reaktor (1) herstellt,
**dadurch gekennzeichnet, dass** das Mittel (11) zur Umlenkung des nach unten durch die Mischkammer strömenden Reaktionsmediums eine Umlenkwanne ist, welche eine in Querrichtung des Reaktors (1) ausgerichtete Fläche, die sich über einen Großteil der Fläche,
die von der unteren Seite des Wärmeüberträgers (2) im Querschnitt des Reaktors (1) eingenommen wird, jedoch nicht über den gesamten Querschnitt des Wärmeüberträgers (2) erstreckt, sowie eine seitliche, sich in Längsrichtung des Reaktors (1) erstreckende Umrandung aufweist, die nach oben, das heißt in Richtung des Ejektors, zeigt, und bis an die Unterseite des Wärmeüberträgers (2) heranreicht, wobei der Ejektor durch eine Treibstrahldüse (9), eine Mischkammer (8), ggf. einen Diffusor (12) und ggf. weitere zylinderförmige Abschnitte (13) gebildet wird.

2. Vorrichtung nach Anspruch 1, wobei der Wärmeübertrager (2) Wärmeübertragerrohre (20) umfasst.

3. Vorrichtung nach Anspruch 1 oder 2, wobei der Wärmeübertrager (2) Wärmeübertragerrohre (20) umfasst und der die Wärmeübertragerrohre (20) umgebende Bereich (21) mit dem Einlass (4) zur Zuführung des Kühlmediums und dem Auslass (5) zur Entnahme des Kühlmediums in Fluidverbindung steht, und der innere Bereich (22) der Wärmeübertragerrohre (20) einen Teil des Reaktionsraums (3) bildet und mit der mindestens einen Treibstrahldüse (9) und dem Auslass (10) zur Entnahme des Reaktionsmediums in Fluidverbindung steht.

4. Vorrichtung nach einem oder mehreren der Ansprüche 1 bis 3, wobei die horizontale Querschnittsfläche der Umlenkwanne von 60 bis 98 % der Fläche beträgt, die durch das untere Ende des Wärmeübertragers (2) im Querschnitt des Reaktors (1) eingenommen wird.

5. Vorrichtung nach einem oder mehreren der Ansprüche 1 bis 4, wobei der Einlass (6) zur Zuführung des gasförmigen Reaktanden in den Reaktionsraum (3) unterhalb des Wärmeübertragers (2) und gleichzeitig oberhalb des Mittels (11) zur Umlenkung des nach unten durch die Mischkammer (8) strömenden Reaktionsmediums angebracht ist.

6. Vorrichtung nach Anspruch 5, wobei zusätzlich ein weiterer Einlass zur Zuführung des gasförmigen Reaktanden in den Reaktionsraum (3) oberhalb des Wärmeübertragers (2) vorgesehen ist.

7. Vorrichtung nach einem oder mehreren der Ansprüche 1 bis 6, wobei die Mischkammer oder die Mischkammern (8) an deren unteren Ende jeweils mit einem Diffusor (12), der durch eine Erweiterung des Querschnitts gegenüber der Mischkammer (8) gebildet wird, physisch verbunden sind.

8. Vorrichtung nach einem oder mehreren der Ansprüche 1 bis 7, wobei jeder in Längsrichtung des Reaktors von oben nach unten angeordneter Ejektor unterhalb der Treibstrahldüse (9) mindestens 3 Abschnitte umfasst, einen oberen Abschnitt, der einen im Wesentlichen runden Querschnitt mit dem Durchmesser d1 aufweist, einen Diffusor (12) als mittleren Abschnitt, dessen Querschnitt sich gegenüber dem oberen Abschnitt von oben nach unten erweitert, und einen unteren Abschnitt, der einen im Wesentlichen runden Querschnitt mit dem Durchmesser d2 aufweist, wobei d2 > d1.

9. Vorrichtung nach Anspruch 8, wobei mehrere Ejektoren verwendet werden.

10. Vorrichtung nach einem oder mehreren der Ansprüche 1 bis 9, wobei als Treibstrahldüsen (9) Mehrlochdüsen verwendet werden.

11. Vorrichtung nach einem oder mehreren der Ansprüche 1 bis 10, wobei für jede Treibstrahldüse (9) mindestens ein Einlass (7) zur Zuführung des flüssigen Reaktanden in den Reaktionsraum (3) vorgesehen ist.

12. Vorrichtung nach einem oder mehreren der Ansprüche 1 bis 11, wobei die Umlenkwanne Entleerungsöffnungen aufweist, die bevorzugt zumindest teilweise verschlossen werden können.

13. Verfahren zur kontinuierlichen Umsetzung von flüssigen Reaktanden mit gasförmigen Reaktanden in einer Vorrichtung gemäß einem oder mehreren der Ansprüche 1 bis 12.

14. Verfahren nach Anspruch 13, wobei als Wärmeübertrager (2) ein Rohrbündelwärmeübertrager verwendet wird und das Reaktionsmedium durch die Wärmeübertragerrohre (20) fließt und das Kühlmedium die Wärmeübertragerrohre (20) umgibt.

15. Verfahren nach Anspruch 13 oder 14, wobei die Umsetzung in Gegenwart eines suspendierten oder gelösten Katalysators erfolgt.

16. Verfahren nach einem oder mehreren der Ansprüche 13 bis 15, wobei der flüssige Reaktand ein Nitroaromat ist und der gasförmige Reaktand Wasserstoff.

17. Verfahren nach einem oder mehreren der Ansprüche 13 bis 16, wobei das Reaktionsprodukt dem externen Kreislauf (15) entnommen wird.

## Claims

1. An apparatus for the continuous reaction of liquids with gases, in particular for the preparation of toluenediamine by hydrogenation of dinitrotoluene, which comprises
- a vertically elongated reactor (1) in which there is a reaction space (3),
- at least one heat exchanger (2) which is arranged within the reactor (1),
- at least one inlet (4) for introducing the cooling medium into the heat exchanger (2),
- at least one outlet (5) for taking the cooling medium off from the heat exchanger (2),
- at least one inlet (6) for introducing the gaseous reactant into the reaction space (3),
- at least one inlet (7) for introducing the liquid reactant into the reaction space (3),
- at least one mixing chamber (8),
- at least one downward-directed driving jet nozzle (9) for introducing the reaction medium, the outlet of which is arranged above the at least one mixing chamber (8) and which is in fluid communication with the reaction space (3),
- at least one outlet (10) for taking the reaction medium off from the reactor and
- at least one means (11) arranged below the heat exchanger (2) and below the mixing chamber (8) for diverting the reaction medium flowing downward through the mixing chamber (8) in such a way that the reaction medium once again flows upward through the heat exchanger (2), and
- where an external circuit (15) having a pump (14) arranged therein provides fluid communication between the outlet (10) for taking off the reaction medium from the reactor (1) and the at least one driving jet nozzle (9) for introduction of the reaction medium into the reactor (1),
- wherein the means (11) for diverting the reaction medium flowing downward through the mixing chamber is a diversion pan which has a surface which is aligned in the transverse direction of the reactor (1) and extends over a major part of the area occupied by the underside of the heat exchanger (2) in the cross section of the reactor (1) but not over the entire cross section of the heat exchanger (2) and also a lateral border which extends in the longitudinal direction of the reactor (1) and points upward, i.e. in the direction of the ejector, and extends to the underside of the heat exchanger (2), where the ejector is formed by a driving jet nozzle (9), a mixing chamber (8), optionally a diffuser (12) and optionally further cylindrical sections (13).

2. The apparatus according to claim 1, wherein the heat exchanger (2) comprises heat exchanger tubes (20).

3. The apparatus according to claim 1 or 2, wherein the heat exchanger (2) comprises heat exchanger tubes (20) and the region (21) surrounding the heat exchanger tubes (20) is in fluid communication with the inlet (4) for introducing the cooling medium and the outlet (5) for taking off the cooling medium and the inner region (22) of the heat exchanger tubes (20) forms part of the reaction space (3) and is in fluid communication with the at least one driving jet nozzle (9) and the outlet (10) for taking off the reaction medium.

4. The apparatus according to one or more of claims 1 to 3, wherein the horizontal cross-sectional area of the diversion pan occupies from 60 to 98% of the area taken up by the lower end of the heat exchanger (2) in the cross section of the reactor (1).

5. The apparatus according to one or more of claims 1 to 4, wherein the inlet (6) for introduction of the gaseous reactant into the reaction space (3) is installed below the heat exchanger (2) and at the same time above the means (11) for diverting the reaction medium flowing downward through the mixing chamber (8).

6. The apparatus according to claim 5, wherein a further inlet for introducing the gaseous reactant into the reaction space (3) is additionally provided above the heat exchanger (2).

7. The apparatus according to one or more of claims 1 to 6, wherein the mixing chamber or the mixing chambers (8) are each physically connected, at their lower end, to a diffusor (12), which is formed by a widening of the cross section compared to the mixing chamber (8).

8. The apparatus according to one or more of claims 1 to 7, wherein each ejector arranged from the top downward in the longitudinal direction of the reactor comprises at least 3 sections underneath the driving jet nozzle (9), namely an upper section which has a substantially round cross section having the diameter d1, a diffusor (12) as middle section, whose cross section widens from the top downward compared to the upper section, and a lower section which has a substantially round cross section having the diameter d2, where d2 > d1.

9. The apparatus according to claim 8, wherein a plurality of ejectors are used.

10. The apparatus according to one or more of claims 1 to 9, wherein multiorifice nozzles are used as driving jet nozzles (9).

11. The apparatus according to one or more of claims 1 to 10, wherein at least one inlet (7) for introducing the liquid reactant into the reaction space (3) is provided for each driving jet nozzle (9).

12. The apparatus according to one or more of claims 1 to 11, wherein the diversion pan has emptying openings which can preferably be at least partially closed.

13. A process for the continuous reaction of liquid reactants with gaseous reactants in an apparatus according to one or more of claims 1 to 12.

14. The process according to claim 13, wherein the heat exchanger (2) used is a shell-and-tube heat exchanger and the reaction medium flows through the heat exchanger tubes (20) and the cooling medium surrounds the heat exchanger tubes (20).

15. The process according to claim 13 or 14, wherein the reaction is carried out in the presence of a suspended or dissolved catalyst.

16. The process according to one or more of claims 13 to 15, wherein the liquid reactant is a nitroaromatic and the gaseous reactant is hydrogen.

17. The process according to one or more of claims 13 to 16, wherein the reaction product is taken off from the external circuit (15).

## Revendications

1. Dispositif pour la mise en réaction continue de liquides avec des gaz, notamment pour la fabrication de toluylène-diamine par hydrogénation de dinitrotoluène, comprenant :
- un réacteur étendu verticalement (1), dans lequel se trouve une chambre de réaction (3),
- au moins un échangeur de chaleur (2), qui est agencé dans le réacteur (1),
- au moins une entrée (4) pour l'introduction du milieu réfrigérant dans l'échangeur de chaleur (2),
- au moins une sortie (5) pour le soutirage du milieu réfrigérant de l'échangeur de chaleur (2),
- au moins une entrée (6) pour l'introduction des réactifs gazeux dans la chambre de réaction (3),
- au moins une entrée (7) pour l'introduction des réactifs liquides dans la chambre de réaction (3),
- au moins une chambre de mélange (8),
- au moins une buse de jet d'entraînement (9) orientée vers le bas pour l'entrée du milieu réactionnel, dont la sortie est agencée au-dessus de ladite au moins une chambre de mélange (8) et qui est raccordée fluidiquement à la chambre de réaction (3),
- au moins une sortie (10) pour le soutirage du milieu de réaction du réacteur, et
- au moins un moyen (11) agencé sous l'échangeur de chaleur (2) et sous la chambre de mélange (8) pour la déviation du milieu réactionnel qui s'écoule vers le bas dans la chambre de mélange (8), de sorte que le milieu réactionnel s'écoule de nouveau vers le haut au travers de l'échangeur de chaleur (2),
- un circuit externe (15) dans lequel est agencé une pompe (14) formant un raccord fluidique entre la sortie (10) pour le soutirage du milieu réactionnel du réacteur (1) et ladite au moins une buse de jet d'entraînement (9) pour l'entrée du milieu réactionnel dans le réacteur (1),
**caractérisé en ce que** le moyen (11) pour la déviation du milieu réactionnel qui s'écoule vers le bas dans la chambre de mélange est un bassin de déviation, qui comprend une surface orientée dans la direction transversale du réacteur (1), qui s'étend sur une grande partie de la surface, qui est occupée par le côté inférieur de l'échangeur de chaleur (2) dans la section transversale du réacteur (1), mais toutefois pas sur l'ensemble de la section transversale de l'échangeur de chaleur (2), ainsi qu'une périphérie latérale qui s'étend dans la direction longitudinale du réacteur (1), qui est orientée vers le haut, c'est-à-dire dans la direction de l'éjecteur et s'étend jusqu'au côté inférieur de l'échangeur de chaleur (2), l'éjecteur étant formé par une buse de jet d'entraînement (9), une chambre de mélange (8),
éventuellement un diffuseur (12) et éventuellement d'autres sections cylindriques (13).

2. Dispositif selon la revendication 1, dans lequel l'échangeur de chaleur (2) comprend des tubes d'échange de chaleur (20).

3. Dispositif selon la revendication 1 ou 2, dans lequel l'échangeur de chaleur (2) comprend des tubes d'échange de chaleur (20) et la zone (21) entourant les tubes d'échange de chaleur (20) est raccordée fluidiquement avec l'entrée (4) pour l'introduction du milieu réfrigérant et la sortie (5) pour le soutirage du milieu réfrigérant, et la zone intérieure (22) des tubes d'échange de chaleur (20) forme une partie de la chambre de réaction (3) et est raccordée fluidiquement avec ladite au moins une buse de jet d'entraînement (9) et la sortie (10) pour le soutirage du milieu de réaction.

4. Dispositif selon une ou plusieurs des revendications 1 à 3, dans lequel la surface de section transversale horizontale du bassin de déviation est de 60 à 98 % de la surface qui est occupée par l'extrémité inférieure de l'échangeur de chaleur (2) dans la section transversale du réacteur (1).

5. Dispositif selon une ou plusieurs des revendications 1 à 4, dans lequel l'entrée (6) pour l'introduction des réactifs gazeux dans la chambre de réaction (3) est disposée sous l'échangeur de chaleur (2) et simultanément au-dessus du moyen (11) pour la déviation du milieu de réaction qui s'écoule vers le bas dans la chambre de mélange (8).

6. Dispositif selon la revendication 5, dans lequel une entrée supplémentaire pour l'introduction des réactifs gazeux dans la chambre de réaction (3) est en outre prévue au-dessus de l'échangeur de chaleur (2).

7. Dispositif selon une ou plusieurs des revendications 1 à 6, dans lequel la chambre de mélange ou les chambres de mélange (8) sont chacune raccordées physiquement à leur extrémité inférieure avec un diffuseur (12), qui est formé par un élargissement de la section transversale par rapport à la chambre de mélange (8).

8. Dispositif selon une ou plusieurs des revendications 1 à 7, dans lequel chaque éjecteur agencé du haut vers le bas dans la direction longitudinale du réacteur comprend sous la buse de jet d'entraînement (9) au moins 3 sections, une section supérieure, qui présente une section transversale essentiellement ronde ayant un diamètre d1, un diffuseur (12) en tant que section centrale, dont la section transversale s'élargit du haut vers le bas par rapport à la section supérieure, et une section inférieure, qui présente une section transversale essentiellement ronde ayant un diamètre d2, avec d2 > d1.

9. Dispositif selon la revendication 8, dans lequel plusieurs éjecteurs sont utilisés.

10. Dispositif selon une ou plusieurs des revendications 1 à 9, dans lequel des buses à plusieurs trous sont utilisées en tant que buses de jet d'entraînement (9).

11. Dispositif selon une ou plusieurs des revendications 1 à 10, dans lequel au moins une entrée (7) pour l'introduction des réactifs liquides dans la chambre de réaction (3) est prévue pour chaque buse de jet d'entraînement (9).

12. Dispositif selon une ou plusieurs des revendications 1 à 11, dans lequel le bassin de déviation comprend des ouvertures de vidage, qui peuvent de préférence être au moins partiellement fermées.

13. Procédé de mise en réaction continue de réactifs liquides avec des réactifs gazeux dans un dispositif selon une ou plusieurs des revendications 1 à 12.

14. Procédé selon la revendication 13, dans lequel un échangeur de chaleur (2) à faisceau de tubes est utilisé en tant qu'échangeur de chaleur et le milieu de réaction s'écoule dans les tubes d'échange de chaleur (20) et le milieu réfrigérant entoure les tubes d'échange de chaleur (20).

15. Procédé selon la revendication 13 ou 14, dans lequel la mise en réaction a lieu en présence d'un catalyseur suspendu ou dissous.

16. Procédé selon une ou plusieurs des revendications 13 à 15, dans lequel le réactif liquide est un composé nitroaromatique et le réactif gazeux est l'hydrogène.

17. Procédé selon une ou plusieurs des revendications 13 à 16, dans lequel le produit de réaction est soutiré du circuit externe (15).
